# EUROPEAN PATENT SPECIFICATION

(11) **EP 2 967 052 B1**
(45) Date of publication and mention of the grant of the patent: **25.12.2019**
(21) Application number: 14764747.3
(22) Date of filing: 17.03.2014
(51) Int. Cl.: A01N 43/16, A01P 21/00

(54) **COMPOSITIONS AND METHODS FOR MODULATING IMMUNITY IN PLANTS**
ZUSAMMENSETZUNGEN UND VERFAHREN ZUR IMMUNMODULATION IN PFLANZEN
COMPOSITIONS ET PROCÉDÉS POUR MODULER L'IMMUNITÉ CHEZ LES VÉGÉTAUX

(30) Priority: 15.03.2013 US 201361789445 P
(43) Date of publication of application: 20.01.2016
(73) Proprietor: Boyce Thompson Institute for Plant Research Inc., Ithaca, NY 14853 (US)
(72) Inventor: KLESSIG, Daniel, F., Dryden, NY 13053 (US); SCHROEDER, France, C., Ithaca, NY 14853 (US); MANOSALVA, Patricia, Ithaca, NJ 14850 (US)
(74) Representative: Potter Clarkson
(86) International application number: PCT/US2014/030136
(87) International publication number: WO 2014/145380

(56) References cited:
- WO-A2-2010/009241
- WO-A2-2010/146062
- WO-A2-2012/084858
- WO-A2-2013/022985
- WO-A2-2013/022997
- US-A1- 2016 037 741
- US-B1- 8 318 146
- FATMA KAPLAN ET AL: "Interspecific Nematode Signals Regulate Dispersal Behavior", PLOS ONE, vol. 7, no. 6, 6 June 2012 (2012-06-06), page e38735, XP055306079, DOI: 10.1371/journal.pone.0038735
- ANDREA CHOE ET AL: "Ascaroside Signaling Is Widely Conserved among Nematodes", CURRENT BIOLOGY, CURRENT SCIENCE, GB, vol. 22, no. 9, 12 March 2012 (2012-03-12) , pages 772-780, XP028483989, ISSN: 0960-9822, DOI: 10.1016/J.CUB.2012.03.024 [retrieved on 2012-03-19]
- JAIME H. NOGUEZ ET AL: "A Novel Ascaroside Controls the Parasitic Life Cycle of the Entomopathogenic Nematode Heterorhabditis bacteriophora", ACS CHEMICAL BIOLOGY, vol. 7, no. 6, 15 June 2012 (2012-06-15), pages 961-966, XP055306189, US ISSN: 1554-8929, DOI: 10.1021/cb300056q
- PATRICIA MANOSALVA ET AL: "Conserved nematode signalling molecules elicit plant defenses and pathogen resistance", NATURE COMMUNICATIONS, vol. 6, 23 July 2015 (2015-07-23), page 7795, XP055305977, DOI: 10.1038/ncomms8795
- GLAZEBROOK J: "Genes controlling expression of defense responses in Arabidopsis-2001 status", 20010801, vol. 4, no. 4, 1 August 2001 (2001-08-01), pages 301-308, XP002205208,

## Description

### FIELD OF THE INVENTION

This invention relates to the fields of agriculture, small molecule pesticides and plant disease resistance. More specifically, the disclosure provides a collection of small molecules called ascarosides and methods of use thereof for modulation of pathogens or resistance to pathogens in a variety of plant species.

### BACKGROUND OF THE INVENTION

Several publications and patent documents are cited throughout the specification in order to describe the state of the art to which this invention pertains.

Over the past two decades, the recognition of specific molecular patterns has been shown to play a central role in the immune responses of plants and animals (Boller and Felix, 2009; Ronald and Beutler, 2010). Plants and animals have been shown to possess pattern recognition receptors that serve to detect several different molecular signatures associated with specific classes of microbes. For example, Arabidopsis recognize bacteria using specific pattern recognition receptors (PRRs) for flagellin, lipopolysaccharide, peptidoglycan, and other pathogen-associated molecular patterns (PAMPs). Perception of PAMPs triggers the initiation of defense responses, which represents the first line of active microbial defense in plants. Additionally, PAMP perception can lead to long-term sensitization of plants, resulting in more rapid and/or more intense activation of future defense responses, which can lead to enhanced resistance to both biotic and abiotic stresses (Conrath et al., 2006). Similar defense responses can be triggered by molecular species originating from the plant itself, so-called damage-associated molecular patterns (DAMPs;(Bianchi, 2007), which, for example, would result from herbivory by insects. In contrast, there are no known conserved insect- or nematode-associated molecular patterns that are recognized by plants, although a few species- or genus-specific families of lipid-derived small molecules from insect oral secretions have been shown to trigger plant defense responses (Schmelz et al., 2009; Schroder, 1998).

Nematodes are arguably the most numerous animals on earth. They are ubiquitous in soil and parasitize most plants and animals, and as a result cause agricultural damage of more than $100 B annually worldwide (Blumenthal and Davis, 2004). In recent work, the Schroeder lab has shown that ascarosides represent an evolutionarily conserved family of nematode-derived small molecules that serve essential functions in regulating development and social behaviors (Choe et al., 2012b; Pungaliya et al., 2009; Srinivasan et al., 2008; von Reuss et al., 2012). Ascarosides are glycosides of the dideoxysugar ascarylose that carry a fatty acid-derived lipophilic side chain and have been identified exclusively from nematodes. Whereas some nematode ascarosides (NAs) are broadly produced among different nematode species, other NAs are highly species-specific or are associated primarily with a specific ecology. For example, the NA ascr#9 is particularly common among entomopathogenic (insect-parasitic) nematodes (Choe et al., 2012b), whereas the longer-chained ascr#18 is produced by several species of the plant-parasitic genus *Meloidogyne* . Different structural variants are often associated with starkly different activity profiles, and biological activity is frequently observed at very low concentrations. We have identified more than 200 different NA structures from over 20 different nematode species, demonstrating that NAs are widely distributed in the nematode phylum, including both human-parasitic and plant-parasitic nematodes (Bose et al., 2012; Choe et al., 2012b; von Reuss et al., 2012). These results indicated that NAs represent a highly conserved molecular signature of nematodes. Based on these results, it seemed possible that NAs are also perceived by the organisms that nematodes interact with, including their plant and animal hosts as well as nematode-associated microorganisms.

### SUMMARY OF THE INVENTION

The invention is as defined in the claims. In accordance with the present disclosure, a method for modulating disease resistance in plants is provided. An exemplary method of the disclosure comprises contacting a plant or plant part with an effective amount of at least one ascaroside, wherein said ascaroside has the formula: 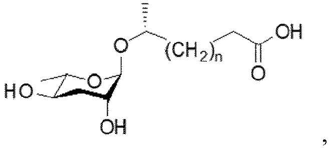 wherein n=7 (ascr# 18), the ascaroside being effective to increase plant resistance to one or more pathogens, and/or inducing one or more plant defense responses, thereby inhibiting pathogen growth and/or infestation, the method may further comprise measuring at least one plant disease response parameter. In a particular embodiment the plant defense response is a basal or innate immune response and is selected from the group consisting of at least one of activation of the systemic acquired resistance, salicylic acid, jasmonate, ethylene, and nitric oxide disease response pathways. In certain instances, the at least one ascaroside is effective to prime a plant defense response. In another embodiment, the plant is contacted with two or more ascarosides and/or with salicylic acid which act additively or synergistically to increase plant pathogen resistance and/or inhibit pathogen growth. The resistance induced may be systemic or localized. Disease response parameters to be assessed in accordance with the method described herein include, but are not limited to: alteration of expression of defense-associated genes, callose deposition, reactive oxygen species production, Ca²⁺ influx, and activation of MAP kinase.

A variety of plants may be treated using the methods disclosed herein. Such plants include, without limitation, tobacco, Arabidopsis, tomato, barley, potato, sweet potato, yam, cotton, soybean, strawberry, sugar beet, corn, rice, wheat, rye, oat, sorghum, millet, bean, pea, apple, banana, pear, cherry, peach, plum, apricot, almond, grape, kiwi, mango, melon, papaya, walnut, hazelnut, pistachio, raspberry, blackberry, loganberry, blueberry, cranberry, orange, lemon, grapefruit, tangerine, lettuce, carrots, onions, broccoli, cabbage, avocado, cocoa, cassava, cotton, and flax.

### BRIEF DESCRIPTION OF THE DRAWINGS

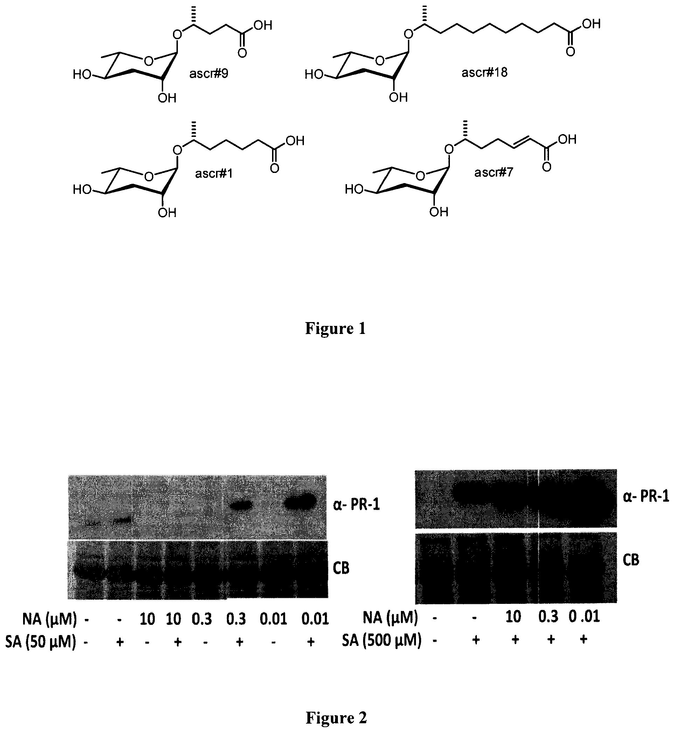

**Figure 1****.** NAs identified in entomopathogenic nematodes (e.g. ascr#9), plant-parasitic nematodes of the genus *Meloidogyne* (ascr#18), and the animal parasites *N. brasiliensis* and *A*. *ceylanicum* (ascr#1 and ascr#7).
**Figure 2****.** Nematode ascaroside ascr#18 enhanced SA induction of PR-1 protein production in tobacco W38 plants. Four-weeks old tobacco plants were syringe-infiltrated with buffer, SA, or a mixture of SA (50 µM or 500 µM) and decreasing concentration of ascr#18 (10, 0.3, and 0.01 µM). PR-1 was assayed 48 hours after treatment by immunoblot using anti-PR-1 antibody. Coomassie Blue (CB) stained loading control indicated that all the samples were equally loaded.

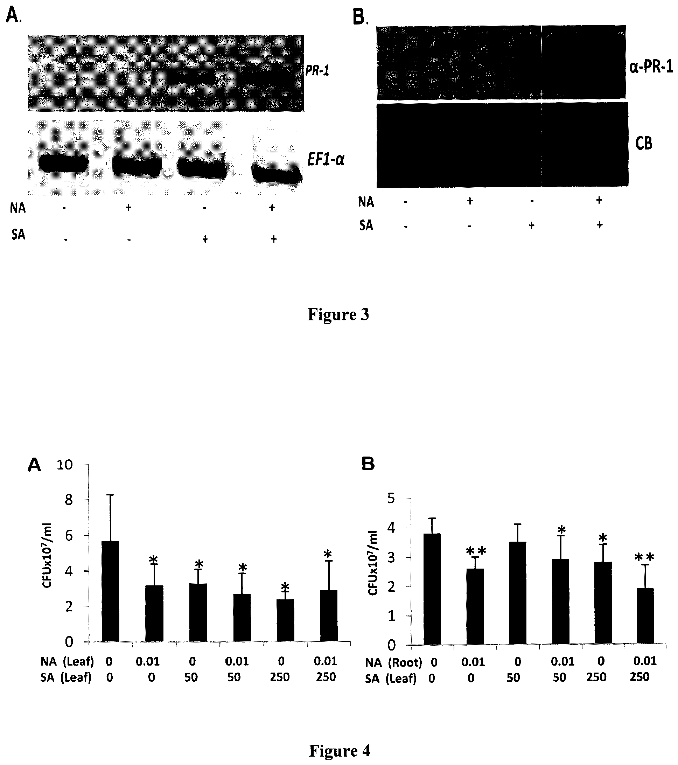

**Figures 3A and 3B****.** Treatment of tobacco roots with ascr#18 enhanced induction by SA of *PR-1* transcript **(****Fig. 3A****)** and protein **(****Fig. 3B****)** in the SA-treated leaves. Roots were treated with NA (0.01 µM) via immersion while the test leaves were simultaneously treated via syringe infiltration with SA (250 µM). *PR-1* expression was analyzed 48 hp SA treatment. *EF1-α* internal control and Coomassie Blue (CB) loading control indicated that all the samples were equally loaded.
**Figures 4A and 4B****.** ascr#18-enhanced resistance in tobacco to virulent *Pseudomonas syringae* pv. *tabaci* (*Pt*). **(****Fig. 4A****)** Leaves were treated by syringe infiltration with ascr#18 (0.01 µM) and/or varying concentrations of SA (50 µM or 250 µM) 24 h before inoculation with *P.t.* **(****Fig. 4B****)** Alternatively, roots were treated via immersion with NA (0.01 µM) while the test leaves were simultaneously treated via syringe infiltration with varying concentrations of SA (50 µM or 250 µM). 24 h after SA treatment leaves were inoculated with *P.t.* Bacterial growth was determined 48 hpi. *P<0.05, **P<0.005 (t-test).

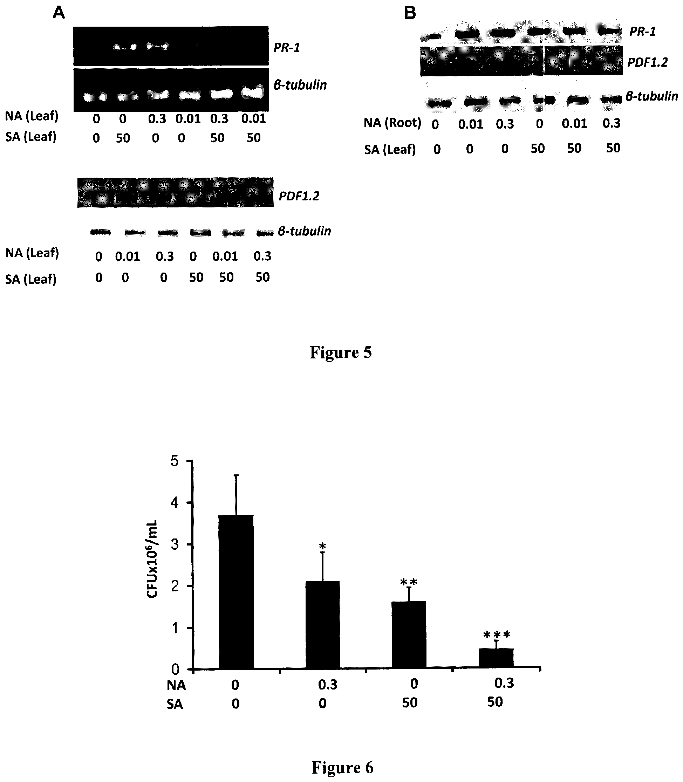

**Figures 5A and 5B****.** ascr#18 altered *PR-1* and *PDF1.2* expression in *Arabidopsis.* **(****Fig. 5A****)** Leaves of four-weeks old *Arabidopsis* ecotype Col-0 were syringe infiltrated with buffer, SA (50 µM), ascr#18 (0.01 or 0.3 µM) or a mixture of SA (50 µM) and ascr#18. *PR-1* and *PDF1.2* expression were detected by semi-quantitative PCR. *β-tubulin* was used as a internal control, which indicated that all the samples were loaded equally. **(****Fig. 5B****)** Alternatively, ascr# 18 was applied to Arabidopsis roots by immersion while the test leaves were simultaneously treated with buffer or SA. Leaves were harvested for RNA 24 h after treatment.
**Figure 6****.** ascr#18-enhanced resistance in *Arabidopsis* to virulent *Pseudomonas syringae* pv. *tomato* DC3000 (*Pst*)*.* Arabidopsis ecotype Col-0 leaves were treated by syringe infiltration with ascr#18 (0.3 µM) and/or SA (50 µM) 24 h prior to inoculation with *Pst.* Bacterial growth was assayed 72 hpi. *P<0.05, **P<0.006, ***P<0.006 (t-test).

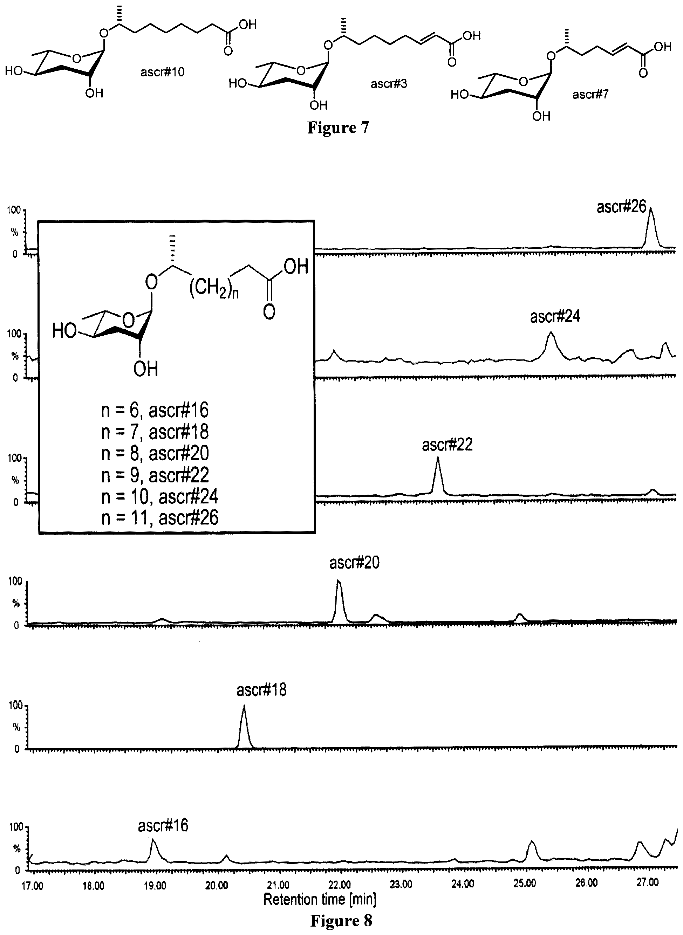

**Figure 7****.** Structures of ascr#10, ascr#3, and ascr#7. Nomenclature for the structures may be found at www.smid-db.org.
**Figure 8****.** NAs identified from *Meloidogyne hapla* infective juveniles. *Nematodes* were incubated in buffer for 24 h and the supernatant collected for analysis by HPLC-MS. Shown are ion chromatograms for ions corresponding to [M-H]⁻ of NAs ascr#16 through ascr#26. In addition, *M. hapla* produces small amounts of the shorter-chained ascr#10.

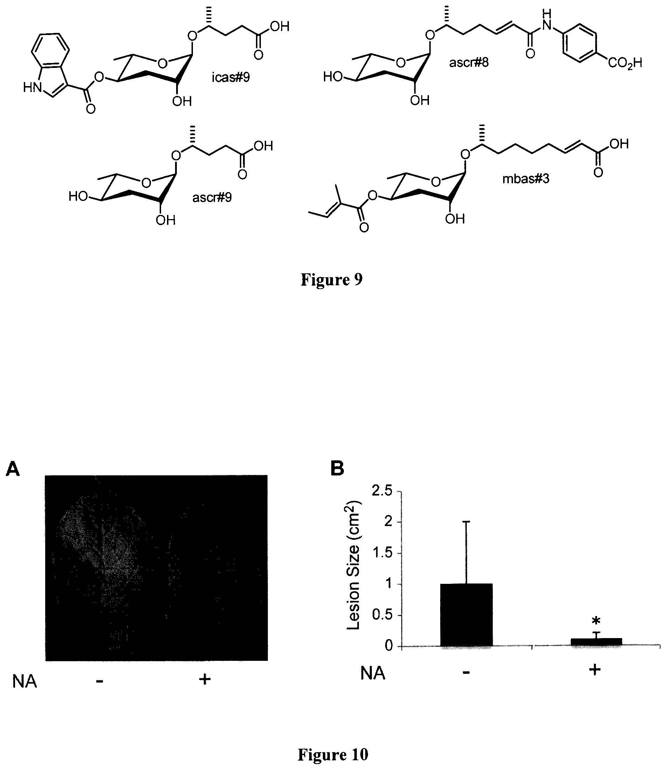

**Figure 9****.** Structures of ascr#9 and NA derivatives icas#9, mbas#3, and ascr#8.
**Figure 10****.** NA enhanced resistance in potato cv. Désirée to virulent US22 strain of *Phytophthera infestans.* Potato plants were treated via root immersion with water (control) or with NA (0.01 µM) 48 h before inoculation with *P. infestans* using a detached leaflet assay. **(A)** Photographs of potato inoculated leaflets 5 dpi. Inoculated area is circled. **(B)** Size of lesion caused by *P. infestans* at 5dpi. * P<0.05 (t-test).

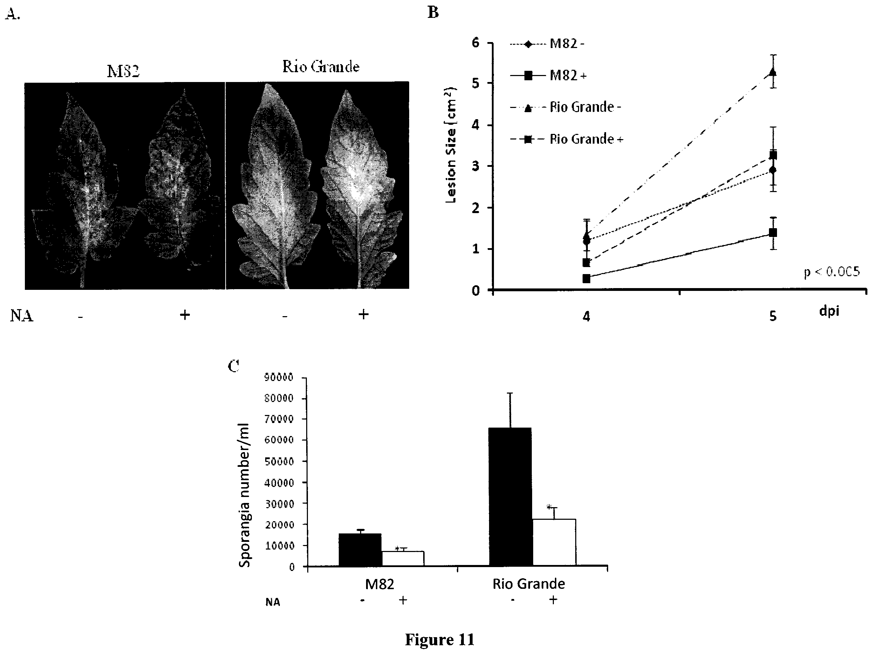

**Figures 11A, 11B****, and 11C.** NA enhanced resistance in tomato cv. M82 and Rio Grande to virulent US22 strain of *P. infestans.* Tomato plants were treated via root immersion with water (-) or with 0.01 µM NA (+) 48 h before inoculation with *P. infestans* using a detached leaflet assay. **Fig. 11A****.** Photographs of tomato inoculated leaflets 6 dpi. **Fig. 11B****.** Size of lesion caused by *P. infestans* at 4 dpi and 5 dpi in the two tomato varieties. **Fig. 11C****.** Sporangia number per ml counted at 6 dpi. * P<0.0005 (t-test).

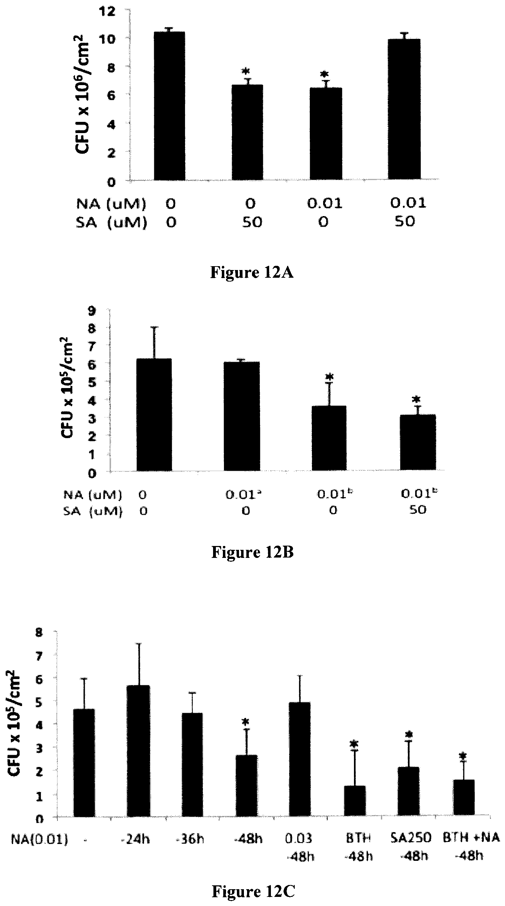

**Figures 12A, 12B, and 12C. Fig. 12A****:** Various methods of treatment with ascr18 enhanced resistance in tobacco to *P. syringae* pv *tobaci.* Tobacco leaves were treated by syringe infiltration with ascr18 (0.01 µM; NA). Salicylic acid (50 µM; SA) was syringe infiltrated in leaves 24 hours after treatment with ascr18. Inoculations of *P. syringae* pv *tabaci* was done 48 hours after ascr18 and bacterial growth was determined at 2 days post inoculation. * P<0.05 (t-test). **Fig. 12B****:** Tobacco plants were sprayed with ascr18 (0.01 µM) 24 hours (a) or 48 hours (b) before inoculation with *P. syringae* pv *tabaci.* * P<0.05 (t-test). **Fig. 12C****:** Roots of tobacco plants were immersed in a solution of ascr18 (0.01 µM or 0.03 µM), SA (250 µM), BTH/actigard (0.075 g/L), or a combination thereof at the indicated times prior to inoculation with *P. syringae* pv *tabaci.* * P<0.05 (t-test).

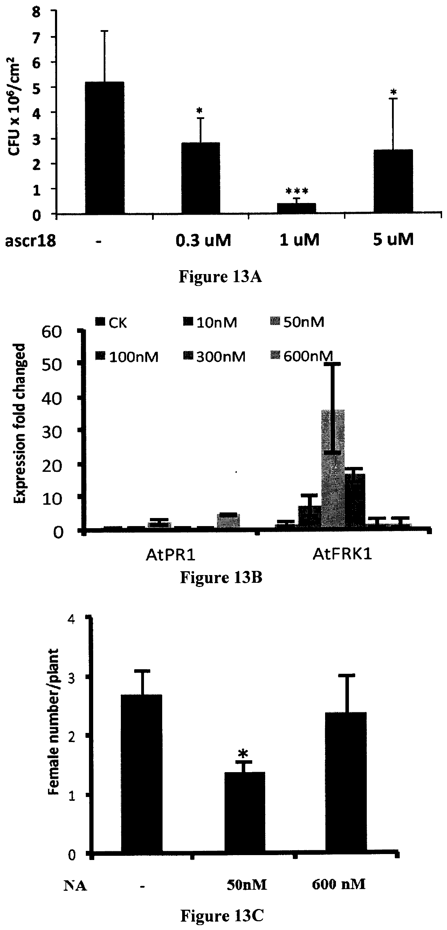

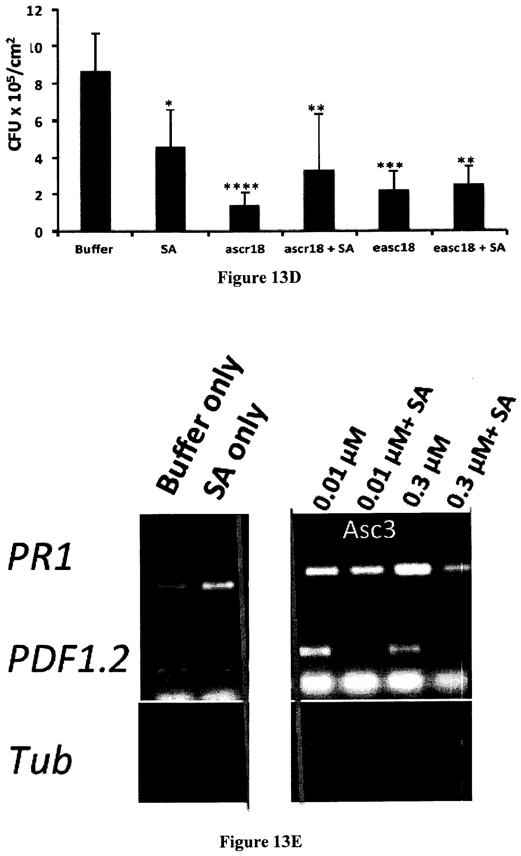

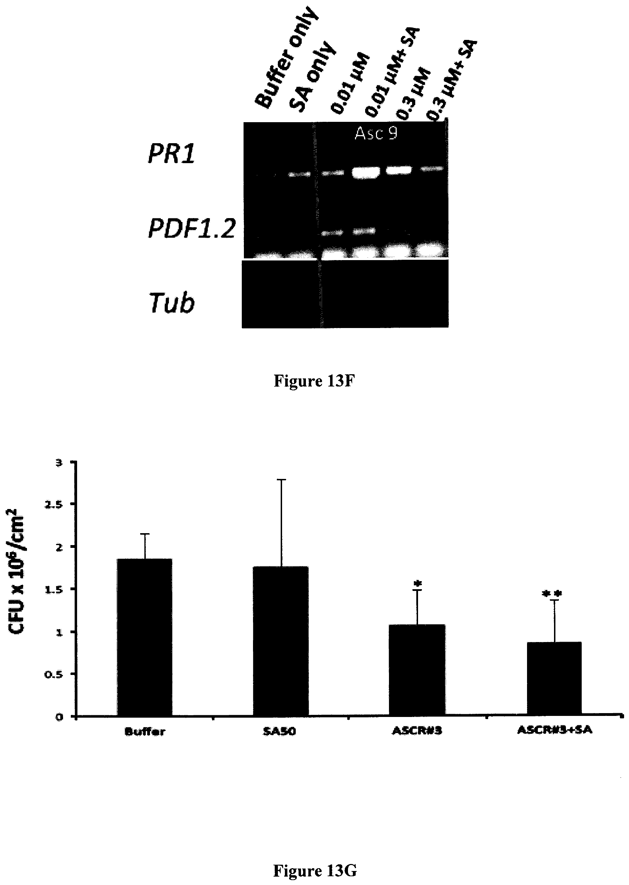

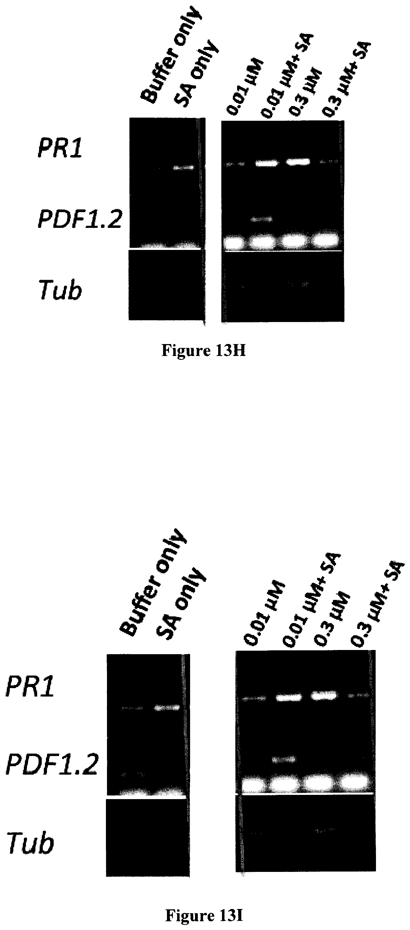

**Figures 13A-13I****.** **Fig. 13A****:** Root treatment with ascr18 enhanced resistance in Arabidopsis to *P. syringae* pv *tomato.* Roots of arabidopsis plants were immersed in a solution of ascr18 (0.3 µM, 1 µM, or 5 µM) 24 hours prior to inoculation with *P. syringae* pv *tomato.* * P<0.05, *** P<0.0005 (t-test). **Fig. 13B****:** Shows the induction of *PR-1* and *FRK1* in ascr18 treated Arabidopsis roots. **Fig. 13C****:** Shows ascr18 enhances resistance of Arabidposis to the cyst nematode *Heterodera schachtii.* * P<0.02 (t-test). **Fig. 13D****:** Treatment with ascr18 enhanced resistance in Arabidopsis to *P. syringae* pv *tomato.* Arabidopsis leaves were treated by syringe infiltration with ascr18 (0.3 µM), easc18 (0.3 µM) and/or SA (50 µM) 24 hours prior to inoculation with *P. syringae* pv *tomato.* Bacterial growth was assayed 3 dpi. *P<0.01, **P<0.001, ***P<0.0001, ****P<0.00005 (t-test). **Fig. 13E****:** Shows ascr3 altered *PR-1* and *PDF1.2* expression in Arabidopsis. 50 µM SA was applied with or without ascr3. Tubulin was used as an internal control. **Fig. 13F****:** Shows ascr9 altered *PR-1* and *PDF1.2* expression in Arabidopsis. 50 µM SA was applied with or without ascr3. Tubulin was used as an internal control. **Fig. 13G****:** Treatment with ascr3 enhanced resistance to *P. syringae* pv *tomato.* Arabidopsis leaves were treated by syringe infiltration with ascr3 (0.3 µM) and/or SA (50 µM) 24 hours prior to inoculation with *P. syringae* pv. *tomato.* Bacterial growth was assayed 3 dpi. *P<0.05, **P<0.001 (t-test). **Fig. 13H****:** Shows ascr10 altered *PR-1* and *PDF1.2* expression in Arabidopsis. Tubulin was used as an internal control. **Fig. 13I****:** Shows oscr9 altered *PR-1* and *PDF1.2* expression in Arabidopsis. Tubulin was used as an internal control.

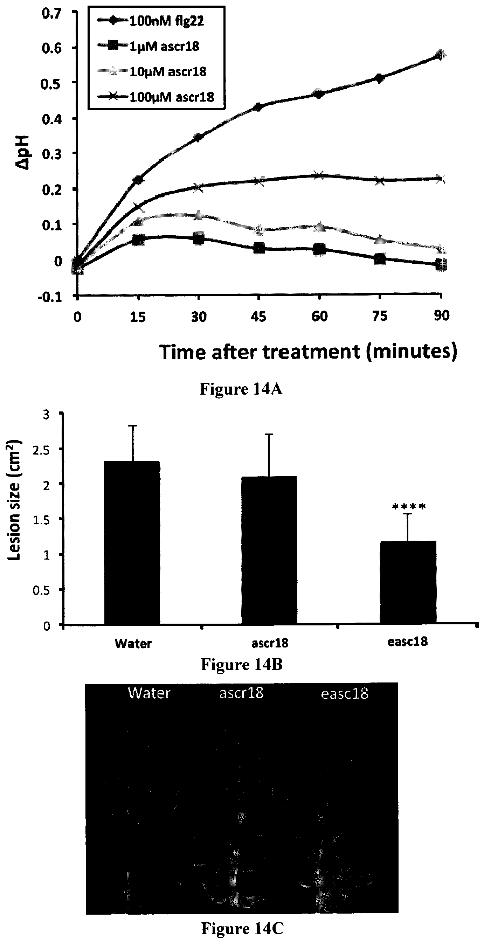

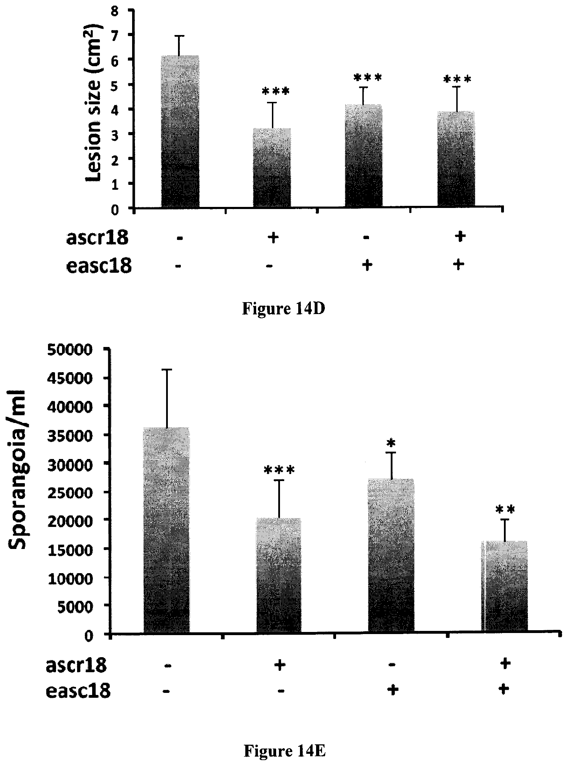

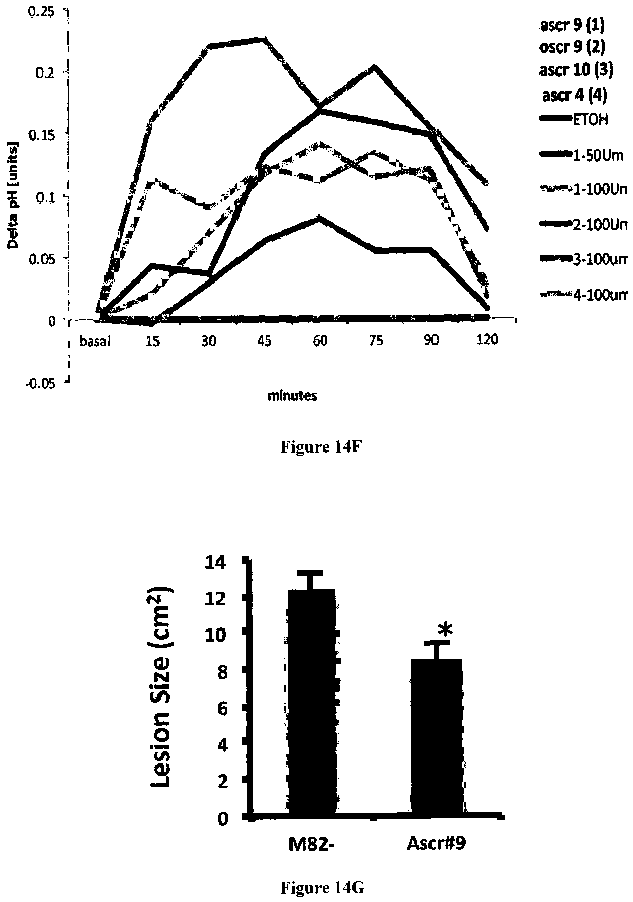

**Figures 14A-14G****.** **Fig. 14A****:** Shows the change in pH of tomato suspension cells treated for 90 minutes with ethanol, Flg22 peptide (positive control), or with the indicated concentrations (µM) of ascr18. **Fig. 14B****:** Treatment with ascr18 enhanced resistance in tomato to *Botrytis cinerea.* Tomato plants were treated via root immersion with water (-) or with 0.01 µM ascr18 or 0.01 µM easc18 48 hours before inoculation with the virulent B05.01 strain of *B. cinerea* using a detached leaflet assay. The size of the lesion caused by B. cinerea was determined at 3 dpi. **Fig. 14C****:** Provides photographs of the *B. cinerea* lesions at 3 dpi. **Fig. 14D****:** Treatment with either ascr18 or easc18 enhanced resistance in tomato to *P. infestans.* Tomato cv. Rio Grande plants were treated via root immersion with water (-) or with 0.01 µM of ascr18 or easc18, or a combination of both 48 hours before inoculation with *P*. *infestans* using a detached leaflet assay. Size of lesion caused by *P. infestans* was determined at 5 dpi. ***P<0.0009 (t-test). **Fig. 14E****:** Sporangia number of *P*. *infestans* was determined at 6 dpi. ***P<0.0009, **P<0.001, *P<0.01 (t-test). **Fig. 14F****:** Tomato suspension cells were treated with either ethanol (ETOH) or ascarosides at the concentration indicated and the increase of the pH in the media was monitored for 120 minutes after adding the ascarosides. **Fig. 14G****:** Tomato cv. M82 plants were treated via root immersion with water (-) or with 1 µM ascr#9 48 hours before inoculation with virulent US22 strain of *Phytophthora infestans* using a detached leaflet assay. Size of lesion caused by *P. infestans* was determined at 7 dpi. *P<0.0005 (t-test).

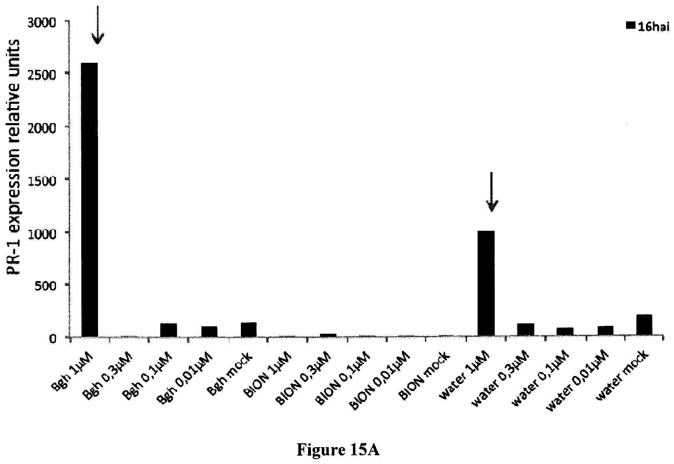

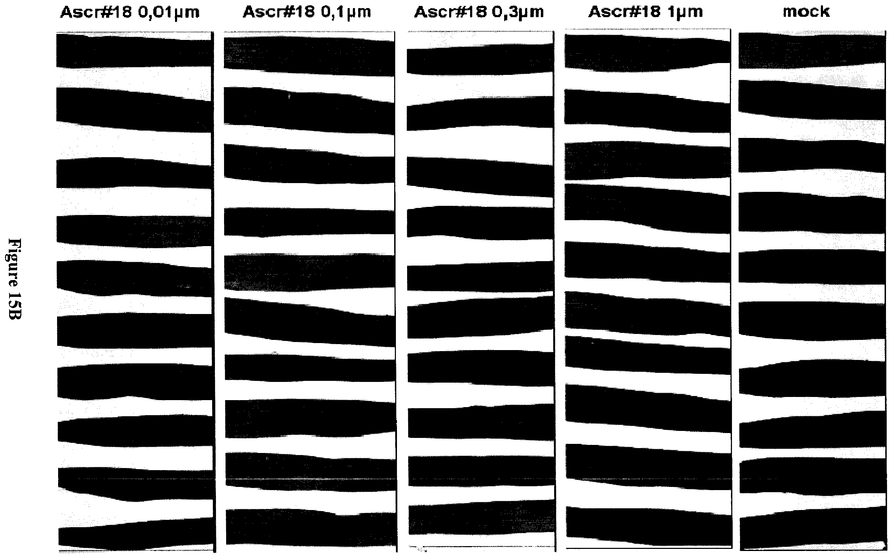

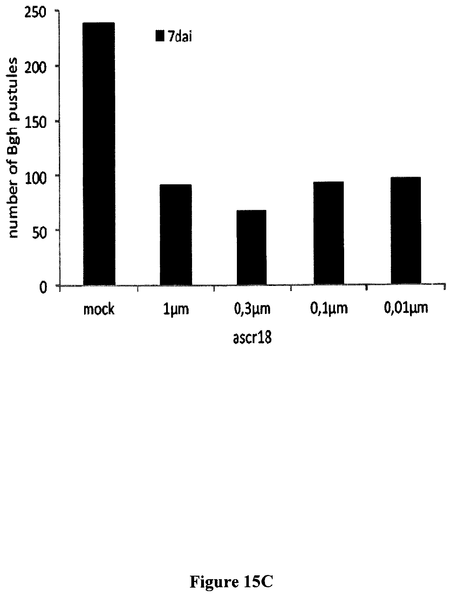

**Figures 15A****,** **15B****, and** **15C****.** **Fig. 15A****:** Shows the induction of PR-1 gene in barley leaves 48 hours after treatment with 1 µM ascr18. **Fig. 15B****:** Treatment with ascr18 of barley leaves enhanced resistance to *Blumeria graminis f.* sp. *hordei* (*Bgh*). Barley leaves were sprayed with the indicated concentrations of ascr18 48 hours before inoculation with *Blumeria graminis f.* sp. *hordei* (*Bgh*). Photograph of barley leave segments infected with *Bgh* were taken at 7 days post inoculation (dpi). **Fig. 15C****:** Provides the number of *Bgh* pustules counted at 7dpi.

### DETAILED DESCRIPTION OF THE INVENTION

Nematode ascarosides (NAs), a highly conserved family of nematode-derived small signaling molecules, act as immunosuppressors in mice and induce morphological changes in fungi that prey on nematodes. The results presented herein indicate that NAs also alter plant defense responses to microbial pathogens. Since nematodes are ubiquitous in soil, they contact virtually all plants via the roots. Identifying the mechanisms by which NAs alter defense responses provides novel insights into plant immunity and facilitates the development of strategies to enhance plant protection against nematodes and other pathogens. Thus, the present invention will also lead to enhanced food security and reduced pesticide use, thereby improving economic and environmental sustainability of agriculture.

A selection of naturally-occurring NA variants as well as additional synthetic variants and derivatives can be synthesized and tested for defense response-modulating activity in tobacco, Arabidopsis, tomato, potato, and other crop plant species, with the most active selected for further development. To further characterize the molecular mechanism(s) by which NAs modulate plant defense responses, several avenues will be explored. Since NA activates salicylic acid (SA)-mediated and jasmonic acid (JA)-mediated defenses and enhances resistance to biotrophic pathogens, NA's signaling mechanism(s) will be investigated using SA-, JA-, and/or ethylene (ET)-defective mutants and global transcriptome analyses. NA's ability to enhance resistance to necrotrophic and biotrophic pathogens, mediated via JA/ET- or SA-dependent pathways, respectively, can be determined, as well as its effect on resistance gene-mediated immunity to microbes and resistance to cyst and root-knot nematodes. To determine whether NAs induce systemic resistance via NA translocation, radiotracer studies can be performed. NA's applicability to multiple crops can be further tested by analyzing defense gene expression and disease resistance.

The following definitions are provided to facilitate an understanding of the present invention.

The term "ascaroside" refers to any of a group of glycolipids, containing the sugar ascarylose, found in most nematode worms.

The term "pathogen" refers to any bacterium, fungus, oomceyte, virus, nematode, or insect, with pathogenic effects on the plant.

The term "pathogen-inoculated" refers to the inoculation of a plant with a pathogen.

The term "disease defense response" refers to a change in metabolism, biosynthetic activity or gene expression that enhances a plant's ability to suppress the replication and spread of a pathogen (i.e., to resist the pathogen). Examples of plant disease defense responses include, but are not limited to, production of low molecular weight compounds with antimicrobial activity (referred to as phytoalexins) and induction of expression of defense (or defense-related) genes, whose products include, for example, peroxidases, cell wall proteins, proteinase inhibitors, hydrolytic enzymes, pathogenesis-related (PR) proteins and phytoalexin biosynthetic enzymes, such as phenylalanine ammonia lyase and chalcone synthase (Dempsey and Klessig, 1995; Dempsey et al., 1999). Such defense responses appear to be induced in plants by several signal transduction pathways involving secondary defense signaling molecules produced in plants. Certain of these defense response pathways are SA dependent, while others are partially SA dependent and still others are SA independent. Agents that are known to induce disease defense responses in plants include, but are not limited to: (1) microbial pathogens, such as fungi, oomycetes, bacteria and viruses and (2) microbial components and other defense response elicitors, such as proteins and protein fragments, small peptides, β-glucans, elicitins, harpins and oligosaccharides. Defense signaling is mediated through several plant hormones, such as SA, ethylene, and jasmonates.

The terms "defense-related genes" and "defense-related proteins" refer to genes or their encoded proteins whose expression or synthesis is associated with or induced after infection with a pathogen.

Treatment of the plants and soil with the ascarosides described herein may be carried out directly or by allowing the compounds to act on the surroundings, environment or storage space by the customary treatment methods, for example by immersion, spraying, evaporation, fogging, scattering, painting on and, in the case of propagation material, in particular in the case of seeds, also by applying one or more coats.

Depending on the plant species or plant cultivars, their location and growth conditions (soils, climate, vegetation period, diet), the treatment according to the invention may also result in super-additive ("synergistic") effects. Thus, for example, reduced application rates and/or a widening of the activity spectrum and/or an increase in the activity of the substances and compositions to be used, better plant growth, increased tolerance to high or low temperatures, increased tolerance to drought or to water or soil salt content, increased flowering performance, easier harvesting, accelerated maturation, higher harvest yields, better quality and/or a higher nutritional value of the harvested products, better storage stability and/or processability of the harvested products that exceed the effects which were actually to be expected may occur.

The ascarosides described herein may be used in unchanged form or together with an agronomically acceptable carrier. The term "agronomically acceptable carrier" includes any carrier suitable for administration to a plant or soil, for example, customary excipients in formulation techniques, such as solutions (e.g., directly sprayable or dilutable solutions), emulsions, (e.g., emulsion concentrates and diluted emulsions), wettable powders, suspensions, soluble powders, powders, dusts, pastes, soluble powders, granules, suspension-emulsion concentrates, encapsulation into polymeric materials, coatable pastes, natural and synthetic materials impregnated with active compound and microencapsulations in polymeric substances. These formulations are produced in a known manner, for example by mixing the compounds with agronomically acceptable carrier, such as liquid solvents or solid carriers, optionally with the use of surfactants, including emulsifiers, dispersants, and/foam-formers.

If the agronomically acceptable carrier is water, it may also possible to employ, for example, organic solvents as auxiliary solvents. Suitable liquid solvents include, for example, aromatics (e.g., xylene, toluene and alkylnaphthalenes); chlorinated aromatics or chlorinated aliphatic hydrocarbons (e.g., chlorobenzenes, chloroethylenes and methylene chloride); aliphatic hydrocarbons (e.g., cyclohexane); paraffins (e.g., petroleum fractions, mineral and vegetable oils); alcohols (e.g., butanol or glycol and also their ethers and esters); ketones (e.g., acetone, methyl ethyl ketone, methyl isobutyl ketone and cyclohexanone) and strongly polar solvents (e.g., dimethylformamide and dimethyl sulphoxide). It is preferred that non toxic carriers be used in the methods of the present invention.

Suitable solid agronomically acceptable carriers include, for example, ammonium salts and ground natural minerals (e.g., kaolins, clays, talc, chalk, quartz, attapulgite, montmorillonite and diatomaceous earth); ground synthetic minerals (e.g., highly disperse silica, alumina and silicates); crushed and fractionated natural rocks (e.g., calcite, marble, pumice, sepiolite and dolomite); synthetic granules of inorganic and organic meals; granules of organic material (e.g., sawdust, coconut shells, maize cobs and tobacco stalks).

Suitable emulsifiers and foam-formers include, for example, nonionic and anionic emulsifiers (e.g., polyoxyethylene fatty acid esters, polyoxyethylene fatty alcohol ethers, for example, alkylaryl polyglycol ethers, alkylsulphonates, alkyl sulphates and arylsulphonates) protein hydrolysates.

Suitable dispersants include, for example, lignin-sulphite waste liquors and ethylcellulose.

Tackifiers such as carboxymethylcellulose and natural and synthetic polymers in the form of powders, granules or latices, such as gum arabic, polyvinyl alcohol and polyvinyl acetate, as well as natural phospholipids, such as cephalins and lecithins, and synthetic phospholipids, can be used in the formulations. Other additives may include, for example, mineral and vegetable oils.

Colorants such as inorganic pigments, for example, iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc may also be included in the agronomically acceptable carrier.

The plant defense inducing compositions may be administered to the plant or soil by any techniques known in the art, including, for example, spraying, atomizing, dusting, scattering, coating or pouring. One of skill in the art would be able to determine the appropriate technique for administration without undue experimentation according the specific pest to be combated, the specific chemical composition and formulation of the compound being employed, the method of applying the compound/formulation, and the locus of treatment.

In one embodiment, the inducers of plant defense responses may be administered by foliar application. In another embodiment, the compositions may also reach the plants through the root system via the soil (systemic action) by drenching the locus of the plant with a liquid preparation or by incorporating the substances into the soil in solid form, e.g., in the form of granules (soil application). In rice cultivations, these granules may be dispensed over the flooded paddy field. The compositions of the invention may also be applied to tubers or seed grain, for example, by soaking, spraying or drenching the seed grain or tubers in a liquid ascaroside containing composition or by coating the tubers or seed grain with a solid ascaroside composition.

The compositions disclosed herein generally comprise between 0.1 and 95% by weight of active compound, preferably between 0.5 and 90%. Favorable application rates are, in general, 0.1 g to 2 kg of active substance (AS) per hectare (ha), for example, 1 g to 1 kg AS/ha or 2 g to 600 g AS/ha. For application of tubers or seed grain, dosages of 1 mg to 1 g active substance per kg of seed grain or tubers may be used.

The term "substantially pure" refers to a preparation comprising at least 50-60% by weight of a given material (e.g.,small molecule, nucleic acid, oligonucleotide, protein, etc.). More preferably, the preparation comprises at least 75% by weight, and most preferably 90-95% by weight of the given compound. Purity is measured by methods appropriate for the given compound (e.g. chromatographic methods, agarose or polyacrylamide gel electrophoresis, HPLC-MS analysis, and the like).

The term "functional" as used herein implies that the ascaroside is functional for the recited assay or purpose, e.g., for modulation of immunity or disease resistance in plants.

Plants and plant cells to be treated using the compositions and methods described herein include, but are not limited to tobacco, Arabidopsis, tomato, barley, potato, sweet potato, yam, cassava, cotton, soybean, strawberry, sugar beet, corn, rice, wheat, rye, oat, sorghum, millet, canola, bean, pea, apple, banana, pear, cherry, peach, plum, apricot, almond, grape, kiwi, mango, melon, papaya, walnut, hazelnut, pistachio, raspberry, blackberry, loganberry, blueberry, cranberry, orange, lemon, grapefruit, tangerine, lettuce, carrots, onions, broccoli, cabbage, avocado, and cocoa.

The ascarosides for use in the methods described herein can vary in structure. The term "alkyl" refers to an aliphatic hydrocarbon group which may be a linear, branched, or cyclic hydrocarbon structure or combination thereof. Representative alkyl groups are those having 24 or fewer carbon atoms, for instance, methyl, ethyl, n-propyl, ipropyl, n-butyl, s-butyl, t-butyl, n-pentyl, i-pentyl, n-hexyl, and the like. Lower alkyl refers to alkyl groups having about 1 to about 6 carbon atoms in the chain. Branched alkyl means that one or more lower alkyl groups such as methyl, ethyl, or propyl are attached to a linear alkyl chain.

The statement that alkyl is intended to include linear, branched, or cyclic hydrocarbon structures and combinations thereof means that an "alkyl" group also includes the following combination of linear and cyclic structural elements 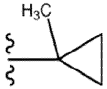 (and similar combinations).

"Alkenyl" means an alkyl, as defined above, containing at least one double bond between adjacent carbon atoms. Alkenyls include both *cis* and *trans* isomers. Branched alkenyl means that one or more lower alkyl groups such as methyl, ethyl, or propyl are attached to a linear alkenyl chain. Representative straight chain and branched alkenyls are those having about 2 to about 6 carbon atoms in the chain, for instance, ethylenyl, propylenyl, I-butenyl, 2-butenyl, isobutylenyl, I-pentenyl, 2-pentenyl, 3-methyl-1-butenyl, 2-methyl-2-butenyl, 2,3-dimethyl-2-butenyl, and the like.

The term "halogen" refers to fluoro, chloro, bromo, and iodo.

The term "halo alkyl" refers to a branched or straight-chain alkyl as described above, substituted with one or more halogens.

The term "haloalkenyl" refers to a branched or straight-chain alkenyl as described above, substituted with one or more halogens.

The term "aryl" means an aromatic monocyclic or multi-cyclic (polycyclic) ring system of 6 to about 19 carbon atoms, for instance, about 6 to about 10 carbon atoms, and includes arylalkyl groups. Representative aryl groups include, but are not limited to, groups such as phenyl, naphthyl, azulenyl, phenanthrenyl, anthracenyl, fluorenyl, pyrenyl, triphenylenyl, chrysenyl, and naphthacenyl.

The term "arylalkyl" means an alkyl residue attached to an aryl ring. Examples are benzyl, phenethyl, and the like.

The term "heteroaryl" means an aromatic monocyclic or multi-cyclic ring system of about 5 to about 19 ring atoms, for instance, about 5 to about 10 ring atoms, in which one or more of the atoms in the ring system is/are element(s) other than carbon, for example, nitrogen, oxygen, and/or sulfur. As is well known to those skilled in the art, heteroaryl rings have less aromatic character than their all-carbon counter parts. Thus, for the purposes of the disclosure, a "heteroaryl" group need only have some degree of aromatic character. For instance, in the case of multi-cyclic ring systems, only one of the rings needs to be aromatic for the ring system to be defined as "heteroaryl". Exemplary heteroaryls contain about 5 to 6 ring atoms. The prefix aza, oxa, thia, or thio before heteroaryl means that at least a nitrogen, oxygen, or sulfur atom, respectively, is present as a ring atom. A nitrogen, carbon, or sulfur atom in the heteroaryl ring may be optionally oxidized; the nitrogen may optionally be quaternized. Representative heteroaryls include, but are not limited to, purinyl, pyridyl, 2-oxo-pyridinyl, pyrimidinyl, pyridazinyl, pyrazinyl, triazinyl, furanyl, pyrrolyl, thiophenyl, pyrazolyl, imidazolyl, oxazolyl, isoxazolyl, thiazolyl, isothiazolyl, triazolyl, oxadiazolyl, thiadiazolyl, tetrazolyl, indolyl, isoindolyl, benzofuranyl, benzothiophenyl, indolinyl, 2-oxoindolinyl, dihydrobenzofuranyl, dihydrobenzothiophenyl, indazolyl, benzimidazolyl, benzooxazolyl, benzothiazolyl, benzoisoxazolyl, benzoisothiazolyl, benzotriazolyl, quinolinyl, isoquinolinyl, quinazolinyl, cinnolinyl, pthalazinyl, quinoxalinyl, and the like.

The terms "cycloalkyl" and "cycloalkenyl" refer to a non-aromatic, saturated (cycloalkyl) or unsaturated (cycloalkenyl), mono- or multi-cyclic ring system of about 3 to about 8 carbon atoms, for instance, about 5 to about 7 carbon atoms. Exemplary cycloalkyl and cycloalkenyl groups include, without limitation, cyclopropyl, cyclobutyl, cyclopentyl, cyclohexyl, cycloheptyl, norbomyl, cyclopropenyl, cyclobutenyl, cyclopentenyl, cyclohexenyl, cyclophenyl, anti-bicyclopropane, syn-tricyclopropane, and the like.

As used herein, "heterocycle" or "heterocyclyl" refers to a stable 3- to 18 membered ring (radical) which is saturated, unsaturated, or aromatic, and which consists of carbon atoms and from one to five heteroatoms selected from the group consisting of nitrogen, oxygen and sulfur. For purposes of this disclosure, the heterocycle may be a monocyclic, bicyclic, or a polycyclic ring system, which may include fused, bridged, or spiro ring systems, including bicyclic rings in which any of the above heterocycles are fused to a benzene ring. The nitrogen, carbon, or sulfur atoms in the heterocycle may be optionally oxidized; the nitrogen atom may be optionally quaternized; and the ring may be partially or fully saturated. The heterocycle may be attached via any heteroatom or carbon atom. Heterocycles include heteroaryls as defined below. Examples of such heterocycles include, without limitation, morpholinyl, pyrrolidinonyl, pyrrolidinyl, piperidinyl, piperizynyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl, and the like. Further heterocycles and heteroaryls are described in Katritzky et al., eds., Comprehensive Heterocyclic Chemistry: The Structure, Reactions, Synthesis and Use of Heterocyclic Compounds, Vol. 1-8, Pergamon Press, N.Y. (1984).

The term "acyl" refers to groups of from 1 to 8 carbon atoms of a straight, branched, or cyclic configuration, saturated, unsaturated, or aromatic, and combinations thereof, attached to the parent structure through a carbonyl functionality. One or more carbons in the acyl residue may be replaced by nitrogen, oxygen, or sulfur as long as the point of attachment to the parent remains at the carbonyl. Examples include acetyl (Ac), benzoyl, propionyl, isobutyryl, t-butoxycarbonyl, benzyloxycarbonyl, and the like.

The term "amino acid" refers to the fragment of an amino acid that remains following amide bond formation via reaction of the amino acid carboxyl group with an amino group of another molecule. The amino acid can be in D- or L-configuration. Suitable amino acids include α-amino acids, β-amino acids, γ-amino acids, δ-amino acids, and ε-amino acids, and include not only natural amino acids (*i.e.,* those found in biological systems, including the twenty amino acids found in natural proteins), but also naturally-occurring variants of such amino acids, as well as synthetic amino acids and their analogues known to those skilled in the art. Exemplary amino acids include the twenty natural amino acids, 4-hydroxyproline, hydroxyysine, demosine , isodemosine, 3-methylhistidine, norvalin, beta-alanine, gamma-aminobutyric acid, citrulline, homocysteine, homoserine, ornithine, and methionine sulfone.

The term "pyrimidine" refers to a heteroaromatic compound containing a benzene ring with two carbon atoms replaced by two nitrogen atoms (diazine). For instance, the following moiety having the carbon atoms at positions 1 and 3 replaced by nitrogen atoms is considered a pyrimidine 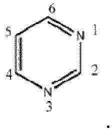 This term, as it is defined herein, also includes its isomeric forms of diazine, such as pyridazine, with the nitrogen atoms in positions 1 and 2; and pyrazine, with the nitrogen atoms in positions 1 and 4. The term "pyrimidine" also generally includes its analogues and derivatives. For instance, the natural nucleobases, cytosine (C), thymine (T), and uracil (D), are pyrimidine derivatives. The term "purine" refers to a heteroaromatic compound containing a pyrimidine ring fused to an imidazole ring. The term "purine" also generally includes its analogues and derivatives. For instance, the natural nucleobases, adenine (A) and guanine (G). Other examples of naturally occuring purine derivatives are hypoxanthine, xanthine, theobromine, caffeine, uric acid, and isoguanine. Exemplary purines and pyrimidines include those disclosed in U.S. Patent No. 3,687,808; Concise Encyclopedia OfPolymer Science And Engineering, pages 858-859, 30 Kroschwitz, 1. 1., ed. John Wiley & Sons, 1990; and Englisch et al., Angewandte Chemie, International Edition, 1991,30,613.

The term "nucleobase" includes all natural and synthetic nucleobases as well as universal nucleobases. Typical natural nucleobases include adenine, guanine, cytosine, uracil, and thymine. Synthetic nucleobases typically include inosine, xanthine, hypoxanthine, nubularine, isoguanisine, or tubercidine. As used herein, a universal nucleobase is any modified, unmodified, naturally occurring or non-naturally occurring nucleobase that can substitute for more than one of the natural nucleobases. Universal bases typically contain an aromatic ring moiety that may, or may not contain nitrogen atoms and generally use aromatic ring stacking to stabilize an oligonucleotide duplex. Some universal bases can be covalently attached to the C-1' carbon of a pentose sugar to make a universal nucleotide. Some universal bases do not hydrogen bond specifically with another nucleobase. Some universal bases base pair with all of the naturally occurring nucleobases. Some universal bases may interact with adjacent nucleotide bases on the same nucleic acid strand by hydrophobic stacking. Exemplary universal nucleobases include, but are not limited to, 2,4-difluorotoluene, nitropyrrolyl, nitroindolyl, 8-aza-7-deazaadenine, 4-fluoro6-methylbenzimidazle, 4-methylbenzimidazle, 3-methyl isocarbostyrilyl, 5-methyl isocarbostyrilyl, 3-methyl-7-propynyl isocarbostyrilyl, 7-azaindolyl, 6-methyl-7-azaindolyl, imidizopyridinyl, 9-methyl-imidizopyridinyl, pyrrolopyrizinyl, isocarbostyrilyl, 7-propynyl isocarbostyrilyl, propynyl-7-azaindolyl, 2,4,5-trimethylphenyl, 4-methylinolyl, 4,6dimethylindolyl, phenyl, napthalenyl, anthracenyl, phenanthracenyl, pyrenyl, stilbenzyl, tetracenyl, pentacenyl, and structural derivatives thereof.

Suitable nucleobases include, but are not limited to, 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2aminopropyl) uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and 0-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine, 7-deazaadenine, N6,N6-dimethyladenine, 2,6-diaminopurine, 5-amino-allyl-uracil, N3-methyluracil, substituted 1,2,4-triazoles, 2-pyridinone, 5-nitroindole, 3-nitropyrrole, 5-methoxyuracil, uracil-5-oxyacetic acid, methoxycarbonylmethyluracil, 5-methyl-2-thiouracil, 5-methoxycarbonylmethyl-2thiouracil, 5-methylaminomethyl-2-thiouracil, 3-(3-amino-3-carboxypropyl)uracil, 3- methylcytosine, 5-methylcytosine, N4-acetyl cytosine, 2-thiocytosine, N6-methyladenine, N6-isopentyladenine, 2-methylthio-N-6-isopentenyladenine, N-methylguanines, and O-alkylated bases. Further purines and pyrimidines include those disclosed in U.S. Pat. No. 3,687,808; Concise Encyclopedia Of Polymer Science And Engineering, pages 858-859; Kroschwitz, 1. 1., ed. John Wiley & Sons, 1990; and Englisch et al., Angewandte Chemie, International Edition, 1991,30,613.

The term "nucleoside" refers to a compound comprising a nucleobase, as defined herein, linked to a pentose at the l'-position. When the nucleobase is a purine derivative or anologue, the pentose is typically attached to the nucleobase at the 9-position of the purine derivative or anologue. When the nucleobase is a pyrimidine derivative or anologue, the pentose is typically attached to the nucleobase at the I-position of the pyrimidine (e.g., Kornberg and Baker, DNA Replication, 2nd Ed., Freeman, San Francisco, 1992). When a nucleoside is present in R³, R⁴, or R⁵ herein, the nucleoside may be connected to the neighboring atom(s) through any atom on the nucleobase or pentose.

The term "fatty acid" generally refers to a carboxylic acid with an aliphatic tail (chain). The aliphatic chain can be between about 2 and about 36 carbon atoms in length. Fatty acids can be saturated, unsaturated, or polyunsaturated. The aliphatic chain can be a linear or a branched chain. The term "fatty acid" may be used herein to refer to a "fatty acid derivative" which can include one or more different fatty acid derivatives, or mixtures of fatty acids derivatives. Exemplary fatty acids include unsaturated fatty acids, saturated fatty acids, and diacids; mono-, di-, and tri-glycerides of ascarosides that have a carboxylic acid functionality; hydroxy acids, co hydroxy acids, co-I hydroxy acids, di-hydroxy fatty acids (e.g., dihydroxy fatty acids that are omega- or omega-l hydroxylated, as well as alpha- or beta-hydroxylated fatty acids).

The term "sugar" refers to a compound which is either a carbohydrate per se made up of one or more monosaccharide units having at least 5 carbon atoms (which may be linear, branched, or cyclic) with an oxygen, nitrogen, or sulfur atom bonded to each carbon atom; or a compound having as a part thereof a carbohydrate moiety made up of one or more monosaccharide units each having at least 5 carbon atoms (which may be linear, branched or cyclic), with an oxygen, nitrogen or sulfur atom bonded to each carbon atom. Representative sugars include the mono-, di-, tri-, and oligosaccharides containing from about 4-9 monosaccharide units, and polysaccharides such as starches, glycogen, cellulose, and polysaccharide gums. Exemplary monosaccharides include C, and above *(e*.*g*.*,* C₅-C₈ or C₅-C₆*)* sugars; di- and trisaccharides include sugars having two or three monosaccharide units .

The term "monosaccharide" means a sugar molecule having a chain of 3-10 carbon atoms in the form of an aldehyde (aldose) or ketone (ketose). Suitable monosaccharides include both naturally occurring and synthetic monosaccharides. Suitable monosaccharides include trioses, such as glycerose and dihydroxyacetone; textroses such as erythrose and erythrulose; pentoses, such as xylose, arabinose, ribose, xylulose ribulose; methyl pentoses (6-deoxyhexoses), such as rhamnose and fucose; hexoses, such as ascarylose, glucose, mannose, galactose, fructose, and sorbose; and heptoses, such as glucoheptose, galamannoheptose, sedoheptulose, and mannoheptulose. Exemplary monosaccharides embrace radicals of allose, altrose, arabinose, cladinose, erythrose, erythrulose, fructose, Dfucitol, L-fucitol, fucosamine, fucose, fuculose, galactosamine, D-galactosaminitol, N-acetyl-galactosamine, galactose, glucosamine, N-acetyl-glucosamine, glucosaminitol, glucose, glucose-6-phosphate, gulose glyceraldehyde, L-glycero-D-mannos-heptose, glycerol, glycerone, gulose, idose, lyxose, mannosamine, mannose, mannose-6-phosphate, psicose, quinovose, quinovasamine, rhamnitol, rhamnosamine, rhamnose, ribose, ribulose, sedoheptulose, sorbose, tagatose, talose, tartaric acid, threose, xylose, and xylulose. The monosaccharide can be in D- or L-configuration. A typical monosaccharide used herein is hexose.

The monosaccharide may further be a deoxy sugar (alcoholic hydroxy group replaced by hydrogen), amino sugar (alcoholic hydroxy group replaced by amino group), a thio sugar (alcoholic hydroxy group replaced by thiol, or CvO replaced by C=S, or a ring oxygen of cyclic form replaced by sulfur), a seleno sugar, a telluro sugar, an aza sugar (ring carbon replaced by nitrogen), an imino sugar (ring oxygen replaced by nitrogen), a phosphano sugar (ring oxygen replaced with phosphorus), a phospha sugar (ring carbon replaced with phosphorus), a C-substituted monosaccharide (hydrogen at a non-terminal carbon atom replaced with carbon), an unsaturated monosaccharide, an alditol (carbonyl group replaced with CHOH group), aldonic acid (aldehydic group replaced by carboxy group), a ketoaldonic acid, a uronic acid, an aldaric acid, and so forth. Amino sugars include amino monosaccharides, such as galactosamine, glucosamine, mannosamine, fucosamine, quinovasamine, neuraminic acid, muramic acid, lactosediamine, acosamine, bacillosamine, daunosamine, desosamine, forosamine, garosamine, kanosamine, kansosamine, mycaminose, mycosamine, perosamine, pneumosamine, purpurosamine, rhodosamine. It is understood that the monosaccharide and the like can be further substituted.

The terms "disaccharide", "trisaccharide", and "polysaccharide" embrace radicals of abequose, acrabose, amicetose, amylopectin, amylose, apiose, arcanose, ascarylose, ascorbic acid, boivinose, cellobiose, cellotriose, cellulose, chacotriose, chalcose, chitin, colitose, cyclodextrin, cymarose, dextrin, 2-deoxyribose, 2-deoxyglucose, diginose, digitalose, digitoxose, evalose, evemitrose, fructoologosachharide, galto-oligosaccharide, gentianose, gentiobiose, glucan, glucogen, glycogen, hamamelose, heparin, inulin, isolevoglucosenone, isomaltose, isomaltotriose, isopanose, kojibiose, lactose, lactosamine, lactosediamine, laminarabiose, levoglucosan, levoglucosenone, ∼-maltose, maltriose, mannan-oligosaccharide, manninotriose, melezitose, melibiose, muramic acid, mycarose, 20 mycinose, neuraminic acid, nigerose, nojirimycin, moviose, oleandrose, panose, paratose, planteose, primeverose, raffinose, rhodinose, rutinose, sarmentose, sedoheptulose, solatriose, sophorose, stachyose, streptose, sucrose, a, a-trehalose, trehalosamine, turanose, tyvelose, xylobiose, umbelliferose, and the like. Further, it is understood that the "disaccharide", "trisaccharide", and "polysaccharide" and the like can be further substituted. Disaccharide also includes amino sugars and their derivatives, particularly, a mycaminose derivatized at the C-4' position or a 4 deoxy-3-amino-glucose derivatized at the C-6' position.

The term "polycyclic" or "multi-cyclic" used herein indicates a molecular structure having two or more rings, including, but not limited to, fused, bridged, or spiro rings.

The above "alkyl", "alkenyl","cycloalkyl", and "cycloalkenyl" radicals, as well as the ring system of the above aryl, heterocyclyl, or heteroaryl groups, may be optionally substituted.

The term "substituted" or "optionally substituted" is used to indicate that a group may have a substituent at each substitutable atom of the group (including more than one substituent on a single atom), provided that the designated atom's normal valency is not exceeded and the identity of each substituent is independent of the others. In accordance with the present disclosure, up to three H atoms in each residue can be replaced with alkyl, halogen, haloalkyl, alkyenyl, haloalkenyl, cycloalkyl, cycloalkenyl, hydroxy, alkoxy, acyl, carboxy, carboalkoxy (also referred to as alkoxycarbonyl), carboxamido (also referred to as alkylaminocarbonyl), cyano, carbonyl, nitro, amino, alkylamino, dialkylamino, mercapto, alkylthio, sulfoxide, sulfone, acylamino, amidino, aryl, heteroaryl, heterocyclyl, aryloxy, heteroaryloxy, a purine or pyridimine or an analogue or derative thereof (as defined in "nucleobase"), or a sugar such as a monosaccharide having 5 or 6 carbon atoms (as defined in "monosaccharide"). "Unsubstituted" atoms bear all of the hydrogen atoms dictated by their valency. When a substituent is keto *(i.e.,* =0), then two hydrogens on the atom are replaced. Combinations of substituents and/or variables are permissible only if such combinations result in stable compounds; by "stable compound" or "stable structure" is meant a compound that is sufficiently robust to survive isolation to a useful degree of purity from a reaction mixture, and formulation into an efficacious agent.

In the characterization of some of the substituents, certain substituents may combine to form rings. Unless stated otherwise, it is intended that such rings may exhibit various degrees of unsaturation (from fully saturated to fully unsaturated), may include heteroatoms, and may be substituted with other substituent groups as described above. The compounds described herein may contain one or more asymmetric centers and may thus give rise to enantiomers, diastereomers, and other stereoisomeric forms. Each chiral center may be defined, in terms of absolute stereochemistry, as (R)- or (S)-. The present disclosure is meant to include all such possible isomers, as well as mixtures thereof, including racemic and optically pure forms. Optically active (R)- and (S)-, (-)- and (+)-, or (D)- and (L)-isomers may be prepared using chiral synthons or chiral reagents, or resolved using conventional techniques. When the compounds described herein contain olefinic double bonds or other centers of geometric asymmetry, and unless specified otherwise, it is intended that the compounds include both E and Z geometric isomers. Likewise, all tautomeric forms are also intended to be included. The configuration of any carbon-carbon double bond appearing herein is selected for convenience only and is not intended to designate a particular configuration; thus a carbon-carbon double bond depicted arbitrarily herein as *trans* may be Z, E, or a mixture of the two in any proportion.

The term "compounds of the invention," and equivalent expressions, are meant to embrace the prodrugs, the pharmaceutically acceptable salts, the oxides, the solvates, e.g. hydrates, and inclusion complexes of that compound, where the context so permits, as well as any stereoisomeric form, or a mixture of any such forms of that compound in any ratio, unless otherwise specified. Inclusion complexes are described in Remington, The Science and Practice of Pharmacy, 19th Ed. 1:176-177 (1995). The most commonly employed inclusion complexes are those with cyclodextrins, and all cyclodextrin complexes, natural and synthetic, are specifically encompassed within the claims. Thus, in accordance with some embodiments of the invention, a compound as described herein, including in the contexts of biologically compatible compositions, methods of treatment, and compounds per se, is provided as the salt form. Similarly, reference to intermediates, whether or not they themselves are claimed, is meant to embrace their salts, and solvates, where the context so permits. For the sake of clarity, particular instances when the context so permits are sometimes indicated in the text, but these instances are purely illustrative and it is not intended to exclude other instances when the context so permits.

The "quaternization" of any basic nitrogen-containing groups of the compounds disclosed herein is also contemplated. The basic nitrogen can be quaternized with any agents known to those of ordinary skill in the art including, for example, lower alkyl halides, such as methyl, ethyl, propyl and butyl chloride, bromides and iodides; dialkyl sulfates including dimethyl, diethyl, dibutyl and diamyl sulfates; long chain halides such as decyl, lauryl, myristyl and stearyl chlorides, bromides and iodides; and aralkyl halides 25 including benzyl and phenethyl bromides. Water or oil-soluble or dispersible products may be obtained by such quaternization.

As used herein, the term "ascaroside" may refer to a compound of Formula I: 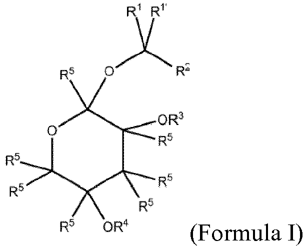 or a pharmaceutical equivalent, derivative, analog, and/or salt thereof. As readily apparent to one of skill in the art, the compound may be further defined by various R groups.

Particular examples of ascarosides for use in the methods of the disclosure, include but are not limited to:

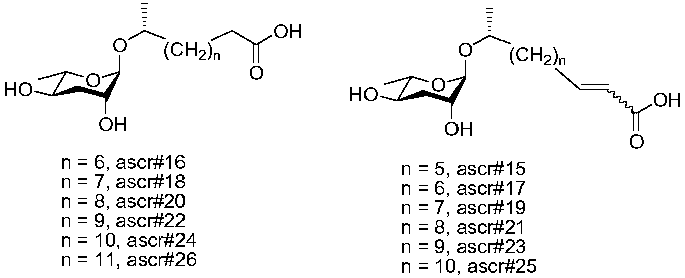

Also contemplated for use in the disclosure are the compounds provided below (see also Figs. 1, 7, and 9), as well as compounds that are structurally identical to the compounds provided below except for the number of carbon atoms in the fatty acid-like side chain (e.g., from between 3 and 24 carbons). Compounds with fatty acid-like side chains containing between 3 and 24 carbon atoms are contemplated for use in the disclosure. 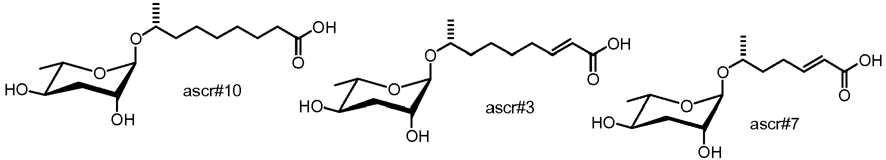 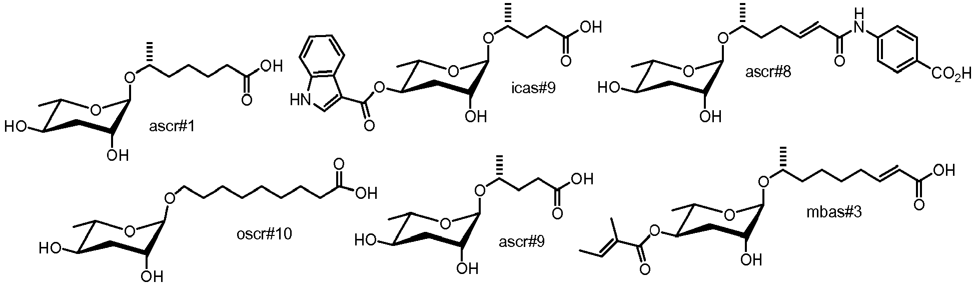 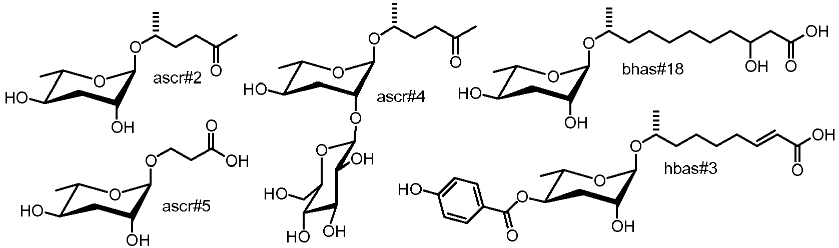 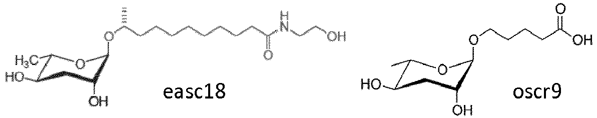 Discriminating non-self from self is a critical aspect of survival that is universal to essentially all living organisms from bacteria, which utilize a restriction-modification system to destroy invading foreign DNA, to vertebrates, which contain a sophisticated, multi-level immune system, including innate immunity and adaptive immunity with B cells, T cells, and accessory cells, that provides exquisite specificity. Plants also have multiple levels of immunity, including non-host resistance, basal resistance, PAMP-triggered immunity (PTI), resistance (R) gene-mediated resistance (also called effector-triggered immunity; ETI), and systemic acquired resistance (SAR). Recognition of foreign compounds by PRRs or R proteins results in significant (often dramatic) alterations in hormonal signaling networks leading to molecular and cellular changes, including callose deposition, reactive oxygen species production, Ca^{2+,} activation of a subset of MAP kinases , and transcriptional reprogramming (Knepper and Day, 2010). These and other changes characterize the immune or defense response in plants.

The following description sets forth the general procedures involved in practicing the present invention. To the extent that specific materials are mentioned, it is merely for purposes of illustration and is not intended to limit the invention. Unless otherwise specified, general biochemical and molecular biological procedures, such as those set forth in Sambrook et al., Molecular Cloning, Cold Spring Harbor Laboratory (1989) (hereinafter "Sambrook et al.") or Ausubel et al. (eds) Current Protocols in Molecular Biology, John Wiley & Sons (1997) (hereinafter "Ausubel et al.") are used.

The following examples are provided to illustrate certain embodiments of the invention. They are not intended to limit the invention in any way.

### EXAMPLE I

### Nematode-derived small molecules can be used as defense-inducing agents to increase crop plant resistance to pathogens

Plants recognize foreign/non-self molecules through pattern-recognition receptors (Boller and Felix, 2009; Knepper and Day, 2010). Several pathogen-associated molecular pattern-containing molecules (PAMPs - also termed MAMPs for microbe-associated molecular patterns) have been identified and shown to induce and/or prime immune responses upon recognition by their cognate receptors. A classic example is the perception of bacterial flagellin by Arabidopsis FLS2. Our initial results indicate that plants recognize nematode ascarosides (NAs) as PAMPs. Thus the present invention provided the means for induction of defense responses using potent, naturally occurring small molecules which will dramatically improve agriculture by facilitating manipulation of disease resistance in crop plants.

### Animals and fungi respond to NAs

Two recent studies have demonstrated that both animals and fungi perceive nanomolar concentrations of NAs. Nematophagous fungi, which are natural predators of soil-dwelling nematodes, use specialized trapping devices to catch and consume nematodes (Barron, 1977). Previous studies demonstrated that most fungal species do not produce traps constitutively, but rather initiate trap formation in response to their prey (Pramer and Stoll, 1959). Co-PD Schroeder, in collaboration with the Sternberg lab, showed that nanomolar concentrations of specific NAs trigger trap formation in nematophagous fungi and that NA-induced morphogenesis is conserved in several closely related species of nematophagous fungi (Hsueh et al., 2012). Specific NAs also have been shown to modulate immune responses in mammals. In collaboration with the Sternberg and Nakayama (Chiba Univ., Japan) groups, the Schroeder lab tested four NAs widely produced by animal-parasitic nematodes and found that NAs ascr#1 and ascr#7 strongly suppressed the development of asthma in a mouse model system.

### NAs modulate plant defense signaling pathways

Nematodes are ubiquitous in soil. Therefore, they are in contact with the roots of virtually all plants. Following plant root colonization, some nematode species cause serious diseases. Annual crop losses worldwide caused by nematodes are estimated to be $100 B (Blumenthal and Davis, 2004) . Other species of nematodes do not cause disease and may even be beneficial. Recent studies revealed that most, if not all, nematode species produce a class of small-molecule signals called ascarosides that elicit specific responses in mammals and fungi.

Previous studies have suggested that plants perceive the presence of nematodes and respond by enhancing their defenses. For example, using a tomato split-root assay, Ogallo and McClure showed that prior inoculation with host-incompatible (avirulent) *Meloidogyne incognita* reduced susceptibility to host-compatible (virulent) *M. hapla* (Ogallo and McClure, 1995).The antagonistic effect of entomopathogenic nematodes on plant-parasitic nematodes (Molina et al., 2007) also may be due to induction of plant defenses, such as *PR-1* gene expression and catalase and peroxidase activity, not only in the roots, but also in the leaves (Jagdale et al., 2009a, b). However, the nature of the nematode-derived signal(s), its perception by the host plant, and the subsequent signaling pathway(s) leading to defense responses has remained a mystery.

Given NAs' effects on animals and fungi, the present inventors initiated an effort to assess NAs' effects, if any, on plant immunity. For this study, we chose to test the NA ascr#18, which is particularly prevalent in several species of the plant parasitic nematode genus *Meloidogyne,* in two plants, tobacco and Arabidopsis.

### Tobacco.

To determine whether ascr#18 affects levels of the prototypic SA-responsive *PR-1* gene/protein, tobacco plants were treated with varying concentrations of ascr#18 in the presence or absence of SA (Figure 2). Application of 0.01, 0.3, or 10 µM ascr#18 alone to leaves did not induce PR-1 protein accumulation. However, in the presence of either high (500 µM) or suboptimal (50 µM) levels of SA, ascr#18 at 0.01 or 0.3 µM enhanced PR-1 accumulation to greater levels than those detected in plants treated only with SA. Interestingly, enhancement was strongest at the lowest concentration of ascr#18, indicating that plants are able to detect NAs at very low concentrations and suggesting that concentrations even lower than 10 nM may be active. By contrast, high levels of ascr#18 (10 µM), appeared to inhibit the ability of 50 µM SA to induce low-level PR-1 accumulation. Biphasic "contradictory" responses to NAs have previously been observed in several studies of nematode chemical communication (Pungaliya et al., 2009; Srinivasan et al., 2008; Srinivasan et al., 2012). The higher induction of *PR-1* expression/protein accumulation in SA and ascr#18 co-treated plants, as compared with SA-treated plants, could be due to a synergistic effect between these compounds or an NA-induced priming effect.

Application of ascr#18 to roots also was found to enhance SA-mediated induction of *PR-1* expression/protein accumulation in tobacco leaves (Figure 3). This result argues that ascr#18 acts systemically. In contrast to the SA-dependent effect of ascr#18 on *PR-1* expression, ascr#18 alone was sufficient to enhance resistance to virulent *Pseudomonas syringae* pv. *tabaci* (*P.t.*) (Figure 4). Leaves or roots treated with NA exhibited similarly reduced levels of virulent *P.t.* growth as leaves treated with 250 µM SA, and no greater reduction was observed when both compounds were provided simultaneously.

### Arabidopsis and other plants.

Analyses of the effects of ascr#18 on Arabidopsis indicate that this species also perceives NA, although its responses differ from those of tobacco. Ascr#18 treatment of test leaves or roots induces *PR-1* expression even in the absence of SA (Figure 5). In addition, ascr#18 treatment induces expression of the prototypic JA-responsive *PDF1.2* gene. Interestingly, ascr#18 suppresses SA-mediated *PR-1* expression, and this effect was observed when NA and SA were co-applied to the test leaves or when ascr#18 was applied through the roots. As in tobacco, pretreatment of Arabidopsis leaves with ascr#18 enhanced resistance to a virulent bacterial pathogen, *P. syringae. pv tomato DC3000* (*Pst*) (Figure 6). Co-treatment with a suboptimal level of SA (50 µM) further enhanced resistance.

Further studies with potato and tomato, two important crop plants, demonstrated the pretreatment of their roots with ascr#18 enhanced resistance to the most devastating plant pathogen, *Phytophthora infestans,* causal agent of late blight and the Great Irish Potato Famine of the 1840s (see Figures 10 and 11).

The observation that NAs modulate plant immune responses provides both important insights into plant immunity and opportunities to enhance plant protection against nematodes and other disease agents. The use of NAs as plant protectants has great potential given that they i) are active at very low concentrations (nM range), ii) can be readily synthesized in large quantities, iii) are bio-degradable, and iv) will face lower regulatory hurdles for approval since they are natural products. Therefore, the present invention will significantly enhance food security worldwide and also reduce the use of chemical pesticides that may be harmful to humans and/or the environment. This novel approach of using small signaling molecules from potential pathogens to prime or activate the plants' immune system will also improve the economic and environmental sustainability of agriculture.

### EXAMPLE II

### NA variants most active in modulating plant defense responses

Bioassays using NAs and nematodes, fungi, or mammals have demonstrated that even minor variations in NA structure strongly affect biological activity (Hsueh et al., 2012; Ludewig and Schroeder, 2013). For example, the seemingly minor difference in the structures of ascr#10 and its unsaturated derivative ascr#3 is associated with profound differences in biological responses of *C*. *elegans:* whereas the hermaphrodite-produced ascr#3 repels hermaphrodites, male-produced ascr#10 strongly attracts hermaphrodites (Izrayelit et al., 2012b). Similarly, the unsaturated ascaroside ascr#7 strongly suppresses ovalbumin-specific T-helper cell responses in mice, whereas ascr#3, which is distinguished from ascr#7 by a two-carbon longer side chain, has no significant effect. Based on these findings, it is likely that ascr#18 is not the only NA affecting plant defense responses and that NAs with more potent or different activity profiles exist. Therefore, we will performed a bioactivity screen of a chemically diverse set of NAs in several plant species that we have shown to respond to ascr#18, such as tobacco, potato, tomato, and Arabidopsis.

### Selection of NAs for bioactivity screening

We focused our initial studies on NAs prevalent among plant-parasitic nematodes, as well as NAs very broadly produced by species from different branches of the nematode phylum (Choe et al., 2012a; Choe et al., 2012b; Izrayelit et al., 2012a; Srinivasan et al., 2012). Representatives of chemically different families of the 200 identified NAs identified to date can be selected and tested for the ability to induce or "prime" an immune or defense resistance response in plants as described herein.

We recently identified the most abundant NAs produced by several species of plant-parasitic nematodes belonging to the genus *Meloidogyne.* HPLC-MS analysis of metabolite extracts obtained from infective juveniles of *M. incognita*, *M. javanica*, and two different *M. hapla* strains consistently revealed abundant production of ascr#16, ascr#18, ascr#20, ascr#22, and ascr#26 in all three species (Figure 8). In addition to assessing the biological activity of these 5 NAs, we will test ascr#10 and its unsaturated derivative ascr#3, as well as the short-chained ascr#9, all of which are very widely produced among parasitic and free-living nematodes (Choe et al., 2012b). Furthermore, we will test the NA derivative icas#9 as it is the most widespread representative of indole ascarosides, and mbas#3 and ascr#8, which are representatives of NA families that are produced abundantly in free-living nematodes including Caenorhabditis spp. (Pungaliya et al., 2009; Srinivasan et al., 2012). Depending on the results from the activity screen of these 10 NAs, we will select up to 10 additional NAs for testing. For example, if the indole ascaroside icas#9 shows high biological activity, we will test additional indole ascarosides, including icas#1, icas#3, and icas#10 (Srinivasan et al., 2012). If short-chained ascarosides such as ascr#9 or ascr#3 show promising activity, we will test additional NAs from this group, which is particularly diverse chemically (von Reuss et al., 2012). Any of the ascarosides disclosed herein may be assessed for modulation of disease resistance in plants.

### Synthesis of NAs for Bioactivity Screening

The chemical synthesis of large varieties of NAs has been worked out in detail and 1-2 g of these compounds can be prepared easily using established methods (Bose et al., 2012; Pungaliya et al., 2009; Srinivasan et al., 2012; von Reuss et al., 2012). Physiological concentrations of NAs in our *M. hapla* cultures have reached up to 100 nM, and many other NAs are found at concentrations up to 10 µM, roughly defining the upper limit of what can be considered a physiological range of concentrations. In soil, NA concentrations are generally much lower. We have found ascr#18 concentrations in the picomolar and low nanomolar range induce a response. Notably, activity was most frequently observed in exactly this concentration range in *C*. *elegans,* nematophagous fungi, and mice. Anticipating the need to provide up to several liters of solution containing NAs at picomolar to low micromolar concentrations for bioassays, we will prepare a multi-gram sample of the NA parent compound, ascarylose, which then will be further modified to produce the different compounds shown in Figures 7-9 using previously described methods (Pungaliya et al., 2009; Srinivasan et al., 2012; von Reuss et al., 2012).

### Bioassays using tobacco and Arabidopsis

Our initial results indicate that ascr#18 induces expression of the SA-regulated *PR-1* and JA-responsive *PDF1.2* genes in Arabidopsis (Figure 5), and enhances SA-induced *PR-1* expression in tobacco (Figures 2 and 3). Thus, the biological activity of the various NAs will initially be assessed by monitoring their ability to (i) induce or enhance expression of *PR-1* in the absence or presence of 50 µM SA in Arabidopsis and tobacco, and (ii) induce expression of *PDF1.2* in Arabidopsis or a yet to be identified JA-responsive gene in tobacco, in which a *PDF1.2* homolog has not been detected. These screens will be performed using 1 µM, 0.01 µM, or 0.0001 µM NA, which will be applied via syringe-infiltration to the leaves. Those NAs with the highest activity will be further tested for their ability to increase resistance to virulent P. *syringae* in both Arabidopsis and tobacco.

We expect that in certain cases, specific structural features of the tested NAs will be associated with particularly strong plant responses. In such cases, additional NA variants will be rationally designed, synthesized and tested for activity. In addition, if several structurally different NAs show significant activity, we will test mixtures of these NAs to assess synergy, as has been documented in both fungi and nematodes (Pungaliya et al., 2009; Srinivasan et al., 2008).

NAs exhibiting the greatest biological activity also can be used for more detailed dose-response analyses of direct or SA-induced *PR-1* expression in Arabidopsis and tobacco. Similar analyses will be performed using *PDF1.2* or an appropriate JA marker gene to assess NA's effect on JA-mediated signaling in Arabidopsis and tobacco, respectively. If one of these NAs is found to be highly active at low nanomolar concentrations, even lower (picomolar and femtomolar) concentrations will be assayed (some NAs have femtomolar activity in *C*. *elegans,* see e.g. (Izrayelit et al., 2012b; Srinivasan et al., 2012; von Reuss et al., 2012)).

### EXAMPLE III

### Characterization of signaling pathways mediating NA responses

The signaling pathways through which the most active NA compounds mediate their effects will be investigated in both tobacco and Arabidopsis. This analysis might be expanded to a second NA, provided it is not only highly active but also elicits a very different effect(s) from the first NA with regard to activation of SA- vs JA-responsive defense genes or enhancement of resistance in Arabidopsis vs tobacco. The selected NAs will be applied to leaves as well as roots to assess whether the results are similar to those obtained following leaf application. In addition, characterizing the NA-mediated systemic signal, as well as assessing NA's effect on plant resistance to nematodes, will involve monitoring plant responses following either leaf or root application.

### Transcriptional profiling of NA responses

For a comprehensive analysis of NA's effect on global gene expression in tobacco and Arabidopsis, RNA-seq analyses (Wang et al., 2009) will be employed. RNA-seq is now the method of choice for transcriptome analysis due to its i) low background noise, ii) dynamic range for quantifying gene expression, iii) ability to distinguish allelic expression and also paralogous expression, iv) low amount of RNA required, and v) relatively low cost. The design of RNA-seq experiments is based on the premise that different NAs will be more active in one plant species than another, and/or that different NAs will elicit distinct phenotypes in different plant species. This possibility is suggested by the finding that a high concentration of ascr#18 (10 µM) inhibited SA-induced *PR-1* expression in tobacco, whereas a 1000-fold lower concentration of this NA (0.01 µM) suppressed SA-induced *PR-1* expression in Arabidopsis. In addition, while low concentrations of ascr#18 (0.01 or 0.3 µM) induced *PR-1* expression in Arabidopsis, they failed to induce PR-1 protein accumulation in tobacco unless SA was provided concurrently. These results suggest that Arabidopsis is more sensitive to ascr#18, with the biphasic dose-response curve shifted such that concentrations that enhance SA-activated *PR-1* expression in tobacco are able to induce defense gene expression on their own in Arabidopsis.

In cases where there are one or two NAs (e.g. ascr#18) that are strongly active in both tobacco and Arabidopsis, we will use those NAs for transcriptional profiling via RNA-seq in both plant species. If it turns out that one of these NAs is more active in Arabidopsis than tobacco, and vice versa, we will perform RNA-seq analyses in both plant species using the corresponding NA with the highest activity in that species. Regardless of whether there are differences in NA activity or the resultant phenotypes in tobacco and Arabidopsis, the parallel transcriptional profiling of both plant species will provide significant advantages. If one or both NAs elicit a similar phenotype in Arabidopsis and tobacco, it is likely that same pathway(s) mediates this response in both species, which may aid with the identification of one or more regulatory hubs affected by NA. Conversely, if one or both NAs have a very different activity level or elicit divergent phenotypes in Arabidopsis and tobacco, combined analysis of the RNA-seq data obtained from both plants will facilitate correlating gene expression changes with the observed differences in response. For all conditions, a minimum of biological triplicates will be used.

For additional transcriptome analyses, NA treatment will be combined with SA or JA treatment. For example, if the most potent NA in Arabidopsis at very low concentrations acts synergistically with SA to induce *PR-1* expression, we will conduct transcriptional profiling after concomitant application of NA and SA at concentrations that result in maximal synergy. To measure NA-modulated changes in pathogen responses, transcriptome analysis also will be performed on NA-treated Arabidopsis before and after infection with *Pst.* Arabidopsis offers several advantages over tobacco because of the very extensive transcriptome databases, particularly those obtained after pathogen infection and/or involving mutants affecting plant immunity. However, as emphasized above, the availability of transcriptomic data in two different species will greatly aid in the interpretation of the RNA-seq results thereby elucidating the general and species-specific effects of NA which will be essential to long-term practical (commercial) application in the field.

### Detailed characterization of pathways involved in NA-induced responses

Results from the transcriptome analyses outlined above should identify the signaling pathways involved in NA responses. Based on our preliminary results, we anticipate that both the SA and JA pathways will be implicated. To confirm their involvement, a two-pronged approach will be use including (i) measuring NA-induced changes in basal pathogen resistance, and (ii) monitoring NA's ability to induce the expression of marker genes, such as *PR-1* and *PDF1.2*, in various defense signaling mutants. Initial experiments with *P. syringae* suggest that treatment with ascr#18 enhances basal resistance to this biotrophic pathogen in both tobacco and Arabidopsis (Figures 4 and 6). These results are consistent with ascr#18's effect on SA-mediated signaling (i.e. induction/enhancement of *PR-1* expression), since resistance to biotrophic pathogens is SA dependent. Induction of *PDF1.2* by ascr#18 in Arabidopsis argues that JA-mediated defense signaling is also affected. Since resistance to necrotrophic pathogens is JA dependent, the effect of NA(s) on basal resistance to necrotrophs, such as *Botrytis cinerea* (or *Alternaria brassicicola*), in Arabidopsis will be tested. The ability of NA(s) to enhance resistance to additional pathogens and/or activate other levels of resistance, including R gene-mediated immunity also will be assessed. For the latter analysis, we will use avirulent *Pst AvrRPS2* or *Pst AvrRPM1* with Arabidopsis, and Tobacco Mosaic Virus (TMV) with the Xanthi nc cultivar of tobacco, which carries the *N* gene and therefore is TMV resistant.

To further investigate the signaling pathways through which NA(s) exerts its effect, NA-mediated induction of *PR-1* and *PDF1.2* and enhanced resistance to *Pst* and *B. cinerea* will be assessed in Arabidopsis mutants compromised for SA-mediated defense signaling (e.g. *isc1* - SA synthesis; *npr1* - SA response) or JA-mediated defense signaling (e.g. *jar1* or *jin1* - JA response). Ethylene (ET) is another important defense signaling hormone that often acts in concert with JA to activate defense genes, such as *PDF1.2*, and resistance against necrotrophs (Ronald and Beutler, 2010). To assess whether NA(s) also utilizes the ET signaling network, NA's ability to induce *PDF1.2* expression and resistance to *B. cinerea* will be monitored in ET mutants (e.g. *etr1* and *ein2* - ET response).

### Assessment of NAs' effects on plant hormone levels

To assess whether the most active NA directly (or indirectly) affects the levels of SA or JA, the levels of these hormones will be quantified before and after *Pst* infection. SA and its glucoside (SAG) will be determined via HPLC, as has been done previously (Liu et al., 2010), while JA levels will be determined by mass spectroscopy (Creelman and Mullet, 1995). Should RNA-seq analysis strongly suggest up or down regulation of genes involved in metabolite production, for example glucosinolate biosynthesis in Arabidopsis or alkaloid (nicotin) biosynthesis in tobacco further metabolome studies can be performed.

### Characterization of the systemic signal in plant NA responses

Our results indicate that NAs can act systemically, since ascr#18 treatment of Arabidopsis or tobacco roots enhanced defense gene expression and resistance to *P. syringae* in the leaves (Figures 3 - 5). Similarly, treating a subset of Arabidopsis leaves with ascr#18 induced *PDF1.2* expression in untreated, as well as treated, leaves. These results suggest that a signal, perhaps the NA itself, moves from the treated roots or leaves to systemic, untreated leaves. Radiolabeled NAs, which can be readily produced via base-catalyzed proton-to-tritium exchange in the side chain, can be used to test whether the NA itself is mobile or instead acts through an unidentified mobile signal to elicit systemic defense responses. Radiolabeled NA will be applied to roots; 24 or 48 hours post treatment, roots and leaves from treated and untreated (control) plants will be homogenized/solubilized and analyzed via scintillation counting. If the results suggest that the NA is mobile, further analyses will be required to determine whether the NA itself or a radiolabeled derivative is translocated to the leaves. For this purpose, we will employ stable-isotope labeling in combination with comparative HPLC-MS. Doubly-deuterium labeled (*d₂*) NA (produced using the same basic protocol as for tritium labeling) and unlabeled NA will be infused into the plant; 24 or 48 hours post treatment, roots and leaves from NA-treated and NA-*d₂*-treated plants will be harvested, extracted with methanol, and analyzed by HPLC-MS/MS using conditions optimized for the detection of ascarosides (von Reuss et al., 2012). HPLC-MS-based comparison of the samples derived from NA-treated and NA-*d₂*-treated plants will reveal any NA derivatives as peaks whose mass increases by 2 mass units in the sample derived from NA-*d₂*-treated plants. If such species are detected, they will be further characterized by high-resolution MS and isolated via preparative HPLC for identification by 2D NMR spectroscopy, as described for example by (Pungaliya et al., 2009).

### Effect of NA application on pathogen resistance in multiple crop species

To test applicability to other crop species, NA-treated tomato, potato, and barley will be analyzed for altered expression of *PR-1* and an appropriate JA-responsive marker gene and enhanced resistance to pathogens. NA's ability to enhance resistance in tomato (both basal and *R* gene-mediated) will be assessed in a *Pto*-carrying cultivar following inoculation with *Pst DC3000* +/- *AvrPto.* Whether NAs enhance resistance in potato and tomato to *Phytophthora infestans*, the causative agent of late blight and the Great Irish Potato Famine of the 1840s, also will be assessed. As part of a large USDA-NIFA funded project on potato and tomato late blight, the Klessig group is currently assessing the role of CRT1 (Kang et al., 2012; Kang et al., 2008; Kang et al., 2010) in basal and *R* gene-mediated resistance to this devastating oomycete pathogen. As a result, all of the cultivars of tomato (+/- the *R* genes *Ph2* and *Ph3*) and potato (+/*RB*) and isolates of *P. infestans* (US11, 22, and 23) that are necessary for investigating NA's effectiveness against this pathogen are available. Early experiments indicate that NA treatment of potato and tomato can dramatically reduce susceptibility to *P. infestans* (Figure 10 and 11A-11C). NA's ability to enhance resistance in barley to *Blumeria graminis* pv. *hordei,* the causative agent of powdery mildew, will also be determined. Ascr18 enhanced resistance to B. graminis in barley (Figures 15B-15C). Assessment of NA's ability to enhance resistance to nematodes will be extended to tomato, potato, and soybean using root-knot nematode (RKN, *Meloidogyne* spp.) and to sugar beet using beet cyst nematode (BCN, *Heterodera schachtii*). Results indicate that ascr18 enhanced resistance in Arabidopsis to H. schachtii (Figure 13C).

### Effects of NAs on plant resistance to nematodes

Previous investigations have shown that prior inoculation with host-incompatible (avirulent) *M. incognita* reduced susceptibility to host-compatible (virulent) *M. hapla* (Ogallo and McClure, 1995). To test whether NAs are responsible for this effect, two or three NAs identified as strong inducers of defense signaling in our initial screen will be assayed in Arabidopsis. Seeds will be germinated on vertical plates with either regular medium or medium containing a selected NA (at nM to µM concentrations); 7-day-old seedlings will be inoculated with surface-sterilized juveniles of BCN (*H. schachtii*) or RKN (*M. incognita*) (Wang et al., 2007). Three-four weeks after nematode inoculation, nematode cyst (for *H. schachtii*) or egg (for *M. incognita*) numbers will be counted and compared with those recovered from control plants to determine if treatment with NAs reduced plant susceptibility to nematode infection.

In addition, nematode infection assays will be conducted on potted plants in the greenhouse. We also will test whether NA treatment alters susceptibility to RKN infection in crop plants such as soybean, tomato, and potato, and to cyst nematode infection in potato. Plants will be grown in 2-inch pots and approximately one week after plant emergence, NAs (at 1 nM to 10 µM concentrations) will be applied to the seedling roots through daily watering for one week. These NA-treated and untreated plants will then be inoculated with RKN or cyst nematode eggs (Chronis et al., 2013; Wang et al., 2007). Four-five weeks after RKN inoculation and 10-12 weeks after cyst nematode inoculation, RKN eggs and cysts will be extracted from plant roots or soil and compared with those recovered from control plants to determine plant susceptibility.

### EXAMPLE IV

### Tobacco

Ascr18 enhances resistance in tobacco against the bacterial pathogen *P. syringae* pv *tobaci* when administered alone (Figure 12A). There was no further enhancement of resistance when salicyclic acid (SA) was added after pretreatment with ascr18. Tobacco leaves were treated by syringe infiltration with ascr18 (0.01 µM). SA (50 µM) was syringe infiltrated in leaves 24 hours after treatment with NA. Bacterial inoculations were done 48 hours after nematode ascaroside (NA) treatment and bacterial growth was assayed at 2 dpi.

Spray application of ascr18 enhances resistance against the bacterial pathogen *P. syringae* pv *tabaci* (Pt) when applied 48 hours but not 24 hours before inoculation (Figure 12B). Spray of a combination of ascr18 and SA did not further increase resistance. Specifically, tobacco plants were sprayed with ascr18 (0.01 µM) 24, 48 hours before inoculation with Pt. SA (50 µM) was syringe infiltrated in leaves 48 hours before inoculation at the time of ascr18 spray. Bacterial growth was assayed at 2dpi.

Treatment by root immersion with ascr18 enhances resistance against the bacterial pathogen *P. syringae* pv *tabaci* (Pt) (Figure 12C). This protection is as efficient as the protection given by root immersion of BTH (Actigard). Roots of tobacco plants were immersed in a solution of the ascr18 at 0.01µM or 0.03 µM, 250 µM SA, 0.075 g/L BTH actigard, or a combination of BTH and ascr18 (0.01 µM) at various times prior to inoculation with Pt. Bacterial growth was assayed at 2 dpi.

### Arabidopsis

Treatment by root immersion with ascr18 enhances resistance against the virulent bacterial pathogen *P. syringae* pv *tomato* (Pst) (Figure 13A). Arabidopsis ecotype Col-0 plants were treated by root immersion with three concentrations of ascr18 (0.3, 1, and 5 µM) 24 hours prior to inoculation with Pst. Bacterial growth was assayed 3 dpi.

Root treatment with ascr18 of Arabidopsis induces PR-1 and the PTI marker gene FRK1 in roots (Figure 13B). Roots of Arabidopsis seedlings were treated with water (CK) or with various ascr18 concentrations for 48 hours. Roots were then collected and used to extract RNA for qRT-PCR analysis.

Ascr18 enhances resistance to cyst nematode *Heterodera schachtii* in Arabidopsis (Figure 13C). Arabidopsis plants growing in media without ascr18 or in media containing 50 nM or 600 nM of ascr18 were inoculated with *H. schachtii* J2s and nematode females were counted at 19 dpi.

Easc18 enhances resistance against virulent *Pseudomonas syringae* pv. *tomato* (Pst) DC3000 (Figure 13D). Arabidopsis ecotype Col-0 leaves were treated by syringe infiltration with ascr18 (0.3 µM), easel 8 (0.3 µM) and/or SA (50 µM) 24 h prior to inoculation with Pst. Bacterial growth was assayed 3 dpi.

Ascr3, ascr9, oscr9, or ascr10 alone induces PR-1 expression in Arabidopsis (Figure 13E, 13F, 13H, and 13I). Ascr3, ascr9, oscr9, or ascr10 applied at low concentrations in combination with SA enhances PR-1 expression induced by SA. However, application of higher concentration of ascr3 or ascr9 reduces the SA-induced PR-1 expression (similar results were obtained in tobacco with ascr3, ascr9, and ascr10, although low concentrations did not significantly increase PR-1 expression tobacco cv. W38). Ascr3, ascr9, or ascr10 alone also induces PDF1.2 expression. Briefly, leaves of four-weeks old plants were syringe infiltrated with buffer, SA (50 µM), ascr3 or ascr9 (0.01 or 0.3 µM) or a mixture of SA (50 µM) and ascr3 or ascr9. PR-1 and PDF1.2 expression were detected by semi-quantitative PCR. β-tubulin was used as an internal control.

Ascr3 enhances resistance against virulent *Pseudomonas syringae* pv. *tomato* (Pst) DC3000 in Arabidopsis (Figure 13G). Ascr9, oscr9, and ascr10 did not significantly enhance resistance against virulent *Pseudomonas syringae* pv. *tomato* (Pst) DC3000 in Arabidopsis at the concentration tested. Arabidopsis ecotype Col-0 leaves were treated by syringe infiltration with ascr3 (0.3 µM) and/or SA (50 µM) 24 hours prior to inoculation with Pst. Bacterial growth was assayed 3 dpi.

### Tomato

Treatment of tomato suspension cells with ascr18 induced media alkalinization (Figure 14A). Accordingly, media alkalinization can be used as a measurement of the NA's bioactivity in tomato. While Ascr18 enhances resistance in tomato cv. M82 and Rio Grande to virulent US22 strain of the oomycete pathogen *Phytophthora infestans*, Ascr18 did not have a significant effect on resistance against the necrotrophic fungal pathogen *Botrytis cinerea* in tomato cv. M82 at the low concentration of 0.01 µM tested. Higher concentrations may prove effective. Notably, ascr3 did not have a significant effect on the resistance of M82 against *Phytophthora infestans* at the low concentration of 0.01 µM tested. Higher concentrations may prove effective.

Easc18 enhances resistance against the virulent necrotrophic fungal pathogen *Botrytis cinerea* in tomato cv. M82 (Figures 14B and Figure 14C). Tomato plants were treated via root immersion with water (-) or with 0.01 µM ascr18 or 0.01 µM easel 8 48 hours before inoculation with the virulent B05.01 strain of *B. cinerea* using a detached leaflet assay.

Easc18 enhances resistance in tomato cv. Rio Grande to virulent US22 strain of the oomycete *P. infestans*, which is comparable with the resistance enhancement by treatment with ascr18 (Figures 14D and Figure 14E). Combination of ascr18 and easc18 did not significantly further enhance resistance to *P. infestans.* Tomato plants were treated via root immersion with water (-) or with 0.01 µM of ascr18, or easc18, or a combination of both NAs (+) 48 hours before inoculation with *P. infestans* using a detached leaflet assay.

Treatment of tomato suspension cells with four new ascarosides induced media alkalinization (Figure 14F). As explained above, media alkalinization may be used as a measurement of ascaroside bioactivity in plants.

Ascr9, though not oscr9 or ascr10 at the concentration tested, enhances resistance against virulent US22 strain of *P. infestans* in tomato cv. M82 as measured by lesion size (Figure 14G) and sporangia number. Tomato plants were treated via root immersion with water (-) or with 1 µM ascr9 48 hours before inoculation with *P. infestans* using a detached leaflet assay. Size of lesion caused by *P. infestans* was determined at 7 dpi.

### Barley

Ascr18 induces PR-1 gene expression in barley at 1µM concentration. Induction is further enhanced by combination of 1µM ascr18 and inoculation with the fungal pathogen *Blumeria graminis* f. sp. *hordei* (*Bgh*) in barley cv. Golden promise (Figure 15A). Leaves of barley plants were sprayed with various concentrations of ascr18 or water (mock) for 48 hours. 48 hours post treatment plants were inoculated with Bgh. Leaves were collected at 16 hpi and used to extract RNA for qRT-PCR analysis.

Ascr18 enhances basal resistance to the fungal pathogen *Blumeria graminis* f. sp. *hordei* (*Bgh*) in barley cv. Golden promise (Figures 15B and 15C). Briefly, barley leaves were sprayed with the indicated concentrations of ascr18 48 hours before inoculation with *Bgh.*

### REFERENCES

Bianchi, M.E. (2007) J Leukocyte Biol 81, 1-5.
Blumenthal, T., and Davis, R.E. (2004) Nat Genet 36, 1246-1247.
Boller, T., and Felix, G. (2009) Annu Rev Plant Biol 60, 379-406.
Bose et al. (2012) Angew Chem Int Ed Engl 51, 12438-12443.
Choe et al. (2012a) Proc Natl Acad Sci U S A 109, 20949-20954.
Choe et al. (2012b) Curr Biol 22, 772-780.
Chronis et al. (2013) Plant J 74:185-96.
Conrath et al. (2006) Molecular Plant-Microbe Interactions 19, 1062-1071.
Creelman, R.A., and Mullet, J.E. (1995) Proc Natl Acad Sci U S A 92, 4114-4119.
Hsueh et al. (2013) Curr Biol 23:83-6.
Izrayelit et al. (2012a) ACS Chem Biol 8:314-9.
Izrayelit et al. (2012b) ACS Chem Biol 7:1321-1325.
Jagdale et al. (2009a) J Nematology 41:341-341.
Jagdale et al. (2009b) Biol Control 51:102-109.
Kang et al. (2012) Nat Commun 3, 1297.
Kang et al. (2008) Cell Host Microbe 3, 48-57.
Kang et al. (2010) Plant Cell 22, 918-936.
Knepper, C., and Day, B. (2010) The Arabidopsis book / Amer Soc Plant Biologists 8, e012.
Liu et al. (2010) Mol Plant Microbe Interact 23, 82-90.
Ludewig, A.H., and Schroeder, F.C. (2013) WormBook, 1-22.
Molina et al. (2007) J Nematol 39, 338-342.
Ogallo, J.L., and McClure, M.A. (1995) J Nematol 27, 441-447.
Oh, C.S., and Martin, G.B. (2011) Trends Plant Sci 16, 132-140.
Pramer, D., and Stoll, N.R. (1959) Science 129, 966-967.
Pungaliya et al. (2009) Proc Natl Acad Sci U S A 106, 7708-7713.
Ronald, P.C., and Beutler, B. (2010). Science 330, 1061-1064.
Schmelz et al. (2009) Proceedings of the National Academy of Sciences 106, 653-657.
Schroder, F. (1998). Angewandte Chemie-International Edition 37, 1213-1216.
Srinivasan et al. (2008) Nature 454, 1115-1118.
Srinivasan et al. (2012) PLoS Biol 10, e1001237.
von Reuss et al. (2012) J Am Chem Soc 134, 1817-1824.
Wang et al. (2007) Molecular Plant Pathology 8, 423-436.
Wang et al. (2009) Nature reviews Genetics 10, 57-63.
Zhong et al. (2011) Cold Spring Harbor protocols 2011, 940-949.

While certain of the preferred embodiments of the present invention have been described and specifically exemplified above, it is not intended that the invention be limited to such embodiments.

## Claims

1. A method for increasing plant resistance to one or more plant pathogens and/or inducing one or more plant defense responses to inhibit plant pathogen growth and/or infestation, said method comprising contacting said plant or plant part with an effective amount of at least one ascaroside, wherein said ascaroside has the formula: 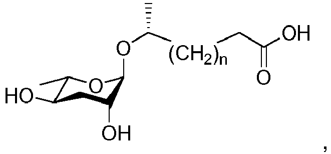 wherein n=7 (ascr#18).

2. The method of claim 1, said method further comprising measuring at least one plant defense response parameter.

3. The method of claim 2, wherein said plant defense response is a basal or innate immune response in the plant or wherein said plant defense response is selected from the group consisting of at least one of activation of the systemic acquired resistance, salicylic acid, jasmonate, ethylene, and nitric oxide disease response pathways.

4. The method of claim 1, wherein said plant defense response parameter is selected from the group consisting of alteration of expression of defense-associated genes such as pathogenesis-related 1 (PR-1) or PDF1.2, callose deposition, reactive oxygen species production, Ca²⁺ influx, and activation of MAP kinase such as mitogen-activated protein kinase 3 (MPK3), MPK4, or MPK6 or their orthologs.

5. The method of claim 1, wherein said plant part is selected from the group consisting of root, stem, leaf, seed and flower.

6. The method of claim 1, wherein said plant is selected from the group consisting of tobacco, Arabidopsis, tomato, barley, potato, sweet potato, yam, cotton, soybean, strawberry, sugar beet, corn, rice, wheat, rye, oat, sorghum, millet, bean, pea, apple, banana, pear, cherry, peach, plum, apricot, almond, grape, kiwi, mango, melon, papaya, walnut, hazelnut, pistachio, raspberry, blackberry, loganberry, blueberry, cranberry, orange, lemon, grapefruit, tangerine, lettuce, carrots, onions, broccoli, cabbage, avocado, cocoa, cassava, cotton, and flax.

7. The method of claim 1, wherein said ascaroside primes an immune response in said plant.

8. The method of claim 1, wherein said contacting results in systemic disease resistance throughout said plant or results in localized resistance in said plant.

9. The method of claim 1, wherein said plant pathogen is a fungus, wherein said plant pathogen is an oomycete, wherein said plant pathogen is a bacterium, wherein said plant pathogen is a nematode, wherein said plant pathogen is a virus, or wherein said plant pathogen is an insect.

10. The method of any one of claims 1-9, wherein said plant is contacted with two or more ascarosides which act additively or synergistically to increase plant pathogen resistance and/or inhibit plant pathogen growth.

11. The method of claim 10, wherein said two ascarosides are ascr#18 and ascr#9, as depicted in Figure 1.

12. The method of claim 1, wherein said plant pathogen is *Pseudomonas syringae* pv. *tabaci,* and wherein said plant is tobacco;
wherein said plant pathogen is *Pseudomonas syringae* pv. *tomato,* and wherein said plant is Arabidopsis;
wherein said plant pathogen is *Phytophthora infestans,* and wherein said plant is potato; or
wherein said plant pathogen is *Phytophthora infestans,* and wherein said plant is tomato.

## Patentansprüche

1. Verfahren zum Erhöhen einer Pflanzenresistenz gegen ein oder mehrere Pflanzenpathogene und/oder Auslösen einer oder mehrerer Pflanzenabwehrreaktionen, um ein Wachstum von und/oder einen Befall durch Pflanzenpathogene zu hemmen, wobei das Verfahren ein Inberührungbringen der Pflanze oder des Pflanzenteils mit einer wirksamen Menge wenigstens eines Akarosids umfasst, wobei das Askarosid die folgende Formel aufweist: 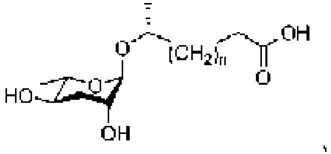 wobei n = 7 (ascr#18).

2. Verfahren nach Anspruch 1, wobei das Verfahren ferner ein Messen wenigstens eines Pflanzenabwehrreaktionsparameters umfasst.

3. Verfahren nach Anspruch 2, wobei die Pflanzenabwehrreaktion eine basale oder angeborene Immunreaktion in der Pflanze ist oder wobei die Pflanzenabwehrreaktion aus der Gruppe ausgewählt ist, die aus einer Aktivierung der systemischen erworbenen Resistenz, Salicylsäure-, Jasmonat-, Ethylen- und/oder Stickoxid-Krankheitsreaktionswegen besteht.

4. Verfahren nach Anspruch 1, wobei der Pflanzenabwehrreaktionsparameter aus der Gruppe ausgewählt ist, die aus Folgendem besteht: einer Abänderung der Expression von Abwehr-assoziierten Genen, wie etwa Pathogenese-bezogenem 1 (PR-1) oder PDF1.2, einer Calloseabscheidung, einer Herstellung reaktiver Sauerstoffspezies, einem Ca²⁺-Zustrom und einer Aktivierung von MAP-Kinase, wie etwa Mitogen-aktivierter Proteinkinase 3 (MPK3), MPK4 oder MPK6 oder deren Orthologen.

5. Verfahren nach Anspruch 1, wobei der Pflanzenteil aus der Gruppe ausgewählt ist, die aus Wurzel, Stamm, Blatt, Samen und Blüte besteht.

6. Verfahren nach Anspruch 1, wobei die Pflanze aus der Gruppe ausgewählt ist, die aus Folgendem besteht: Tabak, Schaumkresse, Tomate, Gerste, Kartoffel, Süßkartoffel, Yamswurzel, Baumwollpflanze, Sojabohne, Erdbeere, Zuckerrübe, Mais, Reis, Weizen, Roggen, Hafer, Sorghum, Rispenhirse, Bohne, Erbse, Apfel, Banane, Birne, Kirsche, Pfirsich, Pflaume, Aprikose, Mandel, Traube, Kiwi, Mango, Melone, Papaya, Walnuss, Haselnuss, Pistazie, Himbeere, Brombeere, Loganbeere, Heidelbeere, Moosbeere, Orange, Zitrone, Grapefruit, Tangerine, Salat, Karotten, Zwiebeln, Brokkoli, Kohl, Avocado, Kakao, Maniok, Baumwollpflanze und Flachs.

7. Verfahren nach Anspruch 1, wobei das Askarosid eine Immunreaktion in der Pflanze fördert.

8. Verfahren nach Anspruch 1, wobei das Inberührungbringen zu einer systemischen Krankheitsresistenz in der gesamten Pflanze führt oder zu einer lokalisierten Resistenz in der Pflanze führt.

9. Verfahren nach Anspruch 1, wobei das Pflanzenpathogen ein Pilz ist, wobei das Pflanzenpathogen ein Eipilz ist, wobei das Pflanzenpathogen ein Bakterium ist, wobei das Pflanzenpathogen ein Nematode ist, wobei das Pflanzenpathogen ein Virus ist, oder wobei das Pflanzenpathogen ein Insekt ist.

10. Verfahren nach einem der Ansprüche 1-9, wobei die Pflanze mit zwei oder mehr Askarosiden in Berührung gebracht wird, die additiv oder synergistisch wirken, um die Pflanzenpathogenresistenz zu erhöhen und/oder das Pflanzenpathogenwachstum zu hemmen.

11. Verfahren nach Anspruch 10, wobei die zwei Askarosiden ascr#18 und ascr#9 sind, wie in Figur 1 dargestellt.

12. Verfahren nach Anspruch 1, wobei das Pflanzenpathogen *Pseudomonas syringae* pv. *tabaci* ist und wobei die Pflanze Tabak ist;
wobei das Pflanzenpathogen *Pseudomonas syringae* pv. *tomate* ist und wobei die Pflanze Schaumkresse ist;
wobei das Pflanzenpathogen *Phytophthora infestans* ist und wobei die Pflanze Kartoffel ist; oder
wobei das Pflanzenpathogen *Phytophthora infestans* ist und wobei die Pflanze Tomate ist.

## Revendications

1. Procédé pour augmenter la résistance des plantes à un ou plusieurs agents phytopathogènes et/ou induire une ou plusieurs réponses de défense de la plante pour inhiber la croissance et/ou l'infestation d'un agent phytopathogène, ledit procédé comprenant la mise en contact de ladite plante ou partie de la plante avec une quantité efficace d'au moins un ascaroside, ledit ascaroside ayant la formule : 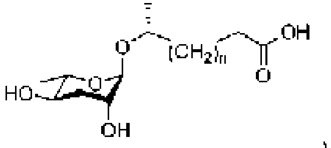 dans laquelle n = 7 (ascr#18).

2. Procédé selon la revendication 1, ledit procédé consistant en outre à mesurer au moins un paramètre de réponse de défense de la plante.

3. Procédé selon la revendication 2, dans lequel ladite réponse de défense de la plante est une réponse immunitaire basale ou innée dans la plante ou dans lequel ladite réponse de défense de la plante est choisie dans le groupe constitué d'au moins une activation de la résistance acquise systémique, de l'acide salicylique, du jasmonate, de l'éthylène et des voies de réponse de la maladie due à l'oxyde nitrique.

4. Procédé selon la revendication 1, dans lequel ledit paramètre de réponse de défense de la plante est choisi dans le groupe constitué d'une altération de l'expression de gènes associés à la défense tels que le gène lié à la pathogénèse 1 (PR-1) ou PDF1.2, d'un dépôt de callose, de la production d'espèces réactives de l'oxygène, de l'influx de Ca²⁺ et de l'activation de la protéine-kinase associée aux membranes telle que la protéine kinase 3 activée par mitogène (MPK3), MPK4 ou MPK6 ou de leurs orthologues.

5. Procédé selon la revendication 1, dans lequel ladite partie de plante est choisie dans le groupe constitué par la racine, la tige, la feuille, la graine et la fleur.

6. Procédé selon la revendication 1, dans lequel ladite plante est choisie dans le groupe constitué par le tabac, l'Arabidopsis, la tomate, l'orge, la pomme de terre, la patate douce, l'igname, le coton, le soja, la fraise, la betterave à sucre, le maïs, le riz, le blé, le seigle, l'avoine, le sorgho, le millet, le haricot, le pois, la pomme, la banane, la poire, la cerise, la pêche, la prune, l'abricot, l'amande, le raisin, le kiwi, la mangue, le melon, la papaye, la noix, la noisette, la pistache, la framboise, la mûre, la mûre de Logan, la myrtille, la canneberge, l'orange, le citron, le pamplemousse, la mandarine, la laitue, les carottes, les oignons, le brocoli, le chou, l'avocat, le cacao, le manioc, le coton et le lin.

7. Procédé selon la revendication 1, dans lequel ledit ascaroside amorce une réponse immunitaire dans ladite plante.

8. Procédé selon la revendication 1, dans lequel ladite mise en contact entraîne une résistance systémique à la maladie dans l'ensemble de ladite plante ou entraîne une résistance localisée dans ladite plante.

9. Procédé selon la revendication 1, dans lequel ledit agent phytopathogène est un champignon, dans lequel ledit agent phytopathogène est un oomycète, dans lequel ledit agent phytopathogène est une bactérie, dans lequel ledit agent phytopathogène est un nématode, dans lequel ledit agent phytopathogène est un virus, ou dans lequel ledit agent phytopathogène est un insecte.

10. Procédé selon l'une quelconque des revendications 1 à 9, dans lequel ladite plante est mise en contact avec au moins deux ascarosides qui agissent de manière additive ou synergique pour augmenter la résistance à un agent phytopathogène et/ou inhiber la croissance d'un agent phytopathogène.

11. Procédé selon la revendication 10, dans lequel lesdits deux ascarosides sont ascr#18 et ascr#9, comme représenté sur la figure 1.

12. Procédé selon la revendication 1, dans lequel ledit agent phytopathogène est *pseudomonas syringae* pv. *tabaci,* et dans lequel ladite plante est du tabac ;
dans lequel ledit agent phytopathogène est *pseudomonas syringae* pv. *tomato,* et dans lequel ladite plante est une Arabidopsis ;
dans lequel ledit agent phytopathogène est *phytophthora infestans* et dans lequel ladite plante est une pomme de terre ; ou
dans lequel ledit agent phytopathogène est *phytophthora infestans* et dans lequel ladite plante est une tomate.
